(19) **Europäisches Patentamt**
**European Patent Office**
**Office européen des brevets**

(11) **EP 2 894 171 A1**

(12) **EUROPEAN PATENT APPLICATION**
published in accordance with Art. 153(4) EPC

(43) Date of publication:
**15.07.2015 Bulletin 2015/29**

(21) Application number: **13834932.9**

(22) Date of filing: **09.09.2013**

(51) Int Cl.:
*C08B 15/10* (2006.01)  *A61K 35/14* (2015.01)
*A61P 3/06* (2006.01)  *A61P 7/04* (2006.01)
*A61P 7/08* (2006.01)  *A61P 9/10* (2006.01)
*A61P 29/00* (2006.01)  *C08B 15/08* (2006.01)

(86) International application number:
**PCT/JP2013/074184**

(87) International publication number:
**WO 2014/038686 (13.03.2014 Gazette 2014/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **10.09.2012 JP 2012198352**

(71) Applicant: **Kaneka Corporation
Osaka-shi, Osaka 530-8288 (JP)**

(72) Inventors:
• **KAWAI, Yoshikazu
Takasago-shi
Hyogo 676-8688 (JP)**
• **MORIO, Ken-ichiro
Takasago-shi
Hyogo 676-8688 (JP)**

• **KONOIKE, Fuminori
Takasago-shi
Hyogo 676-8688 (JP)**
• **WATANABE, Kana
Settsu-shi
Osaka 566-0072 (JP)**
• **SUZUKI, Yasuyuki
Takasago-shi
Hyogo 676-8688 (JP)**
• **HIRANO, Masaru
Takasago-shi
Hyogo 676-8688 (JP)**

(74) Representative: **Vossius & Partner
Patentanwälte Rechtsanwälte mbB
Siebertstrasse 3
81675 München (DE)**

(54) **ADSORBENT**

(57)     The objective of the present invention is to obtain an adsorbent having high adsorption capacity and high strength comprising porous cellulose beads obtained without using an auxiliary material which is highly toxic and corrosive and without a cumbersome and industrially adverse step. The present invention is characterized by immobilizing a ligand onto porous cellulose beads obtained by mixing a cold alkaline aqueous solution and cellulose powder as a raw material to prepare a cellulose dispersion and bringing the cellulose dispersion into contact with a coagulating solvent.

[Fig.1]

EP 2 894 171 A1

SEQUENCE LISTING

<110>   KANEKA CORPORATION

<120>   ADSORBENT

<130>   Y1362 EP

<140>   EP 13 83 4932.9
<141>   2013-09-09

<150>   JP 2012-198352
<151>   2012-09-10

<160>   2

<170>   PatentIn version 3.5

<210>   1
<211>   291
<212>   PRT
<213>   Staphylococcus aureus

<400>   1

Ala Gln His Asp Glu Ala Gln Gln Asn Ala Phe Tyr Gln Val Leu Asn
1               5                   10                  15

Met Pro Asn Leu Asn Ala Asp Gln Arg Asn Gly Phe Ile Gln Ser Leu
            20                  25                  30

Lys Asp Asp Pro Ser Gln Ser Ala Asn Val Leu Gly Glu Ala Gln Lys
        35                  40                  45

Leu Asn Asp Ser Gln Ala Pro Lys Ala Asp Ala Gln Gln Asn Lys Phe
        50                  55                  60

Asn Lys Asp Gln Gln Ser Ala Phe Tyr Glu Ile Leu Asn Met Pro Asn
65                  70                  75                  80

Leu Asn Glu Glu Gln Arg Asn Gly Phe Ile Gln Ser Leu Lys Asp Asp
                85                  90                  95

Pro Ser Gln Ser Thr Asn Val Leu Gly Glu Ala Lys Lys Leu Asn Glu
            100                 105                 110

Ser Gln Ala Pro Lys Ala Asp Asn Asn Phe Asn Lys Glu Gln Gln Asn
            115                 120                 125

Ala Phe Tyr Glu Ile Leu Asn Met Pro Asn Leu Asn Glu Glu Gln Arg
            130                 135                 140

Asn Gly Phe Ile Gln Ser Leu Lys Asp Asp Pro Ser Gln Ser Ala Asn
145                 150                 155                 160

```
Leu Leu Ala Glu Ala Lys Lys Leu Asn Glu Ser Gln Ala Pro Lys Ala
                165             170             175

Asp Asn Lys Phe Asn Lys Glu Gln Gln Asn Ala Phe Tyr Glu Ile Leu
            180             185             190

His Leu Pro Asn Leu Asn Glu Glu Gln Arg Asn Gly Phe Ile Gln Ser
            195             200             205

Leu Lys Asp Asp Pro Ser Gln Ser Ala Asn Leu Leu Ala Glu Ala Lys
    210             215             220

Lys Leu Asn Asp Ala Gln Ala Pro Lys Ala Asp Asn Lys Phe Asn Lys
225             230             235             240

Glu Gln Gln Asn Ala Phe Tyr Glu Ile Leu His Leu Pro Asn Leu Thr
            245             250             255

Glu Glu Gln Arg Asn Gly Phe Ile Gln Ser Leu Lys Asp Asp Pro Ser
            260             265             270

Val Ser Lys Glu Ile Leu Ala Glu Ala Lys Lys Leu Asn Asp Ala Gln
    275             280             285

Ala Pro Lys
    290


<210>  2
<211>  216
<212>  PRT
<213>  artificial

<220>
<223>  orientation-controlled protein A

<400>  2

Ala Asp Asn Arg Phe Asn Arg Glu Gln Gln Asn Ala Phe Tyr Glu Ile
1               5               10              15

Leu His Leu Pro Asn Leu Thr Glu Glu Gln Arg Asn Ala Phe Ile Gln
            20              25              30

Ser Leu Arg Asp Asp Pro Ser Val Ser Arg Glu Ile Leu Ala Glu Ala
        35              40              45

Arg Arg Leu Asn Asp Ala Gln Ala Pro Arg Ala Asp Asn Arg Phe Asn
    50              55              60
```

```
Arg Glu Gln Gln Asn Ala Phe Tyr Glu Ile Leu His Leu Pro Asn Leu
65              70              75              80

Thr Glu Glu Gln Arg Asn Ala Phe Ile Gln Ser Leu Arg Asp Asp Pro
            85              90              95

Gln Ala Pro Arg Ala Asp Asn Arg Phe Asn Arg Glu Gln Gln Asn Ala
            100             105             110

Phe Tyr Glu Ile Leu His Leu Pro Asn Leu Thr Glu Glu Gln Arg Asn
        115             120             125

Ala Phe Ile Gln Ser Leu Arg Asp Asp Pro Ser Val Ser Arg Glu Ile
        130             135             140

Leu Ala Glu Ala Arg Arg Leu Asn Asp Ala Gln Ala Pro Arg Ala Asp
145             150             155             160

Asn Arg Phe Asn Arg Glu Gln Gln Asn Ala Phe Tyr Glu Ile Leu His
            165             170             175

Leu Pro Asn Leu Thr Glu Glu Gln Arg Asn Ala Phe Ile Gln Ser Leu
            180             185             190

Arg Asp Asp Pro Ser Val Ser Arg Glu Ile Leu Ala Glu Ala Arg Arg
            195             200             205

Leu Asn Asp Ala Gln Ala Pro Lys
            210             215
```

**Description**

TECHNICAL FIELD

The present invention relates to an adsorbent. Specifically, the present invention relates to the adsorbent having high adsorption capacity, comprising porous cellulose beads obtained by a given product method.

BACKGROUND ART

Adsorbents using porous cellulose beads have advantages that safety is higher than other synthetic polymers and non-specific adsorption properties are low, a usable pH range is wide, and mechanical strength is large while adsorbents are made from polysaccharides. Such adsorbents are exemplified by adsorbents for various medical treatments, various chromatographies and an affinity chromatography. Among these, adsorbents for affinity chromatography are used as adsorbents for medical treatment and adsorbents for purifying an antibody medical drug, since a target substance can be efficiently purified and a concentration of an unwanted substance can be decreased by using the adsorbents. In particular, an adsorbent obtained by immobilizing protein A as an affinity ligand on a porous support is attracting attention as a therapeutic (medical) adsorbent for arthritis, hemophilia and dilated cardiomyopathy (For example, Non-Patent Document 1 and Non-Patent Document 2). On the other hand, an adsorbent (adsorbent for purifying antibody medical drug) obtained by immobilizing protein A as an affinity ligand on a porous support is also attracting attention as an adsorbent for specifically adsorbing and eluting immunoglobulin (IgG). In addition, an adsorbent for treating high plasma cholesterol in which dextran sulfate is bonded to porous cellulose beads is commercially available (for example, Liposorber manufactured by KANEKA CORPORATION). In the preparation of porous cellulose beads used in adsorbents, since there is little solvent capable of dissolving cellulose powder as raw materials, cellulose is dissolved in a solvent such as aqueous calcium thiocyanate solution having high corrosiveness and toxicity as well as high degree of difficulty to construct facilities which are used for the preparation, and is coagulated to prepare porous cellulose beads (for example, Patent Document 1). On the other hand, there is a method for producing a porous cellulose support by introducing substituents to improve a solubility of cellulose into hydroxy groups of cellulose, dissolving the cellulose in a general solvent, granulating the cellulose, and then removing the substituents (for example, Patent Document 2). However, the method has complicated steps, and a molecular weight of cellulose is decreased during the steps of introducing and removing the substituents. Therefore, the produced support is inclined not to have sufficient strength required for high speed process or large scale procedure, which is recently needed.

As a solvent which can easily dissolve cellulose, an ionic liquid is attracting attention. Non-Patent Document 3 discloses a method for obtaining cellulose beads by dissolving cellulose in an ionic liquid. However, the ionic liquid is not suited for being used as an auxiliary material in industrial level, since the ionic liquid is considerably expensive. In addition, with respect to the safety of the ionic liquid which would remain even in a slight amount, there is only a few toxicity data and the like thereof. Further, when the ionic liquid is used for medical purpose or for producing an adsorbent to purify pharmaceutical compounds, it is predictable that confirmation of the safety of the ionic liquid is considerably required.

PRIOR ART

PATENT DOCUMENT

   Patent Document 1: US 2009/0062118
   Patent Document 2: WO 2006/025371

NON-PATENT DOCUMENT

   Non-Patent Document 1: Annals of the New York Academy of Sciences 2005. Vol. 1051 p. 635-646
   Non-Patent Document 2: American Heart Journal Vol. 152, Number 4 2006
   Non-Patent Document 3: Journal of Chromatography A, 1217 (2010) 1298-1304

## SUMMARY OF THE INVENTION

### PROBLEM TO BE SOLVED BY THE INVENTION

The objective of the present invention is to provide an adsorbent having high adsorption capacity, using porous cellulose beads having high mechanical strength obtained by a simple method without using an auxiliary material which is highly toxic and corrosive in the light of the problems for conventional techniques.

### MEANS FOR SOLVING THE PROBLEMS

As a result of intensive studies in order to solve the above problems, the present inventors have found that, in the preparation of porous cellulose beads, porous cellulose beads are obtained by bringing the cellulose dispersion into contact with a coagulating solvent in a state that cellulose powder as raw materials is not completely dissolved in a cold alkaline aqueous solution, and an adsorbent using the porous cellulose beads has specifically high adsorption capacity, to complete the present invention. That is, an adsorbent of the present invention is characterized by using porous cellulose beads obtained by mixing a cold alkaline aqueous solution and cellulose powder as raw materials to prepare a cellulose dispersion, and bringing the cellulose dispersion into contact with a coagulating solvent.

The adsorbent of the present invention preferably comprises an affinity ligand. An introduced amount of the affinity ligand is, for example, not less than 1 mg and not more than 500 mg per 1 mL of the adsorbent.

The affinity ligand is preferably protein A in some cases.

It is preferable that the protein A has an alkaline resistance.

Further, it is preferable that the protein A is an orientation-controlled protein A.

For example, when the adsorbent having protein A as a ligand adsorbs IgG, it is preferable that 5 % DBC of IgG for residence time of 3 minutes is not less than 60 g/L.

In addition, it is preferable that 5 % DBC of IgG for residence time of 6 minutes is not less than 70 g/L.

The present invention relates to an adsorbent comprising dextran sulfate as a ligand.

For example, when the adsorbent having dextran sulfate as a ligand adsorbs LDL cholesterol, it is preferable that the adsorption capacity of LDL cholesterol is not less than 7 g/L.

When the porous cellulose beads used in the present invention are obtained, the temperature of the cold alkaline aqueous solution is preferably not more than 20 °C. A cellulose concentration in the cellulose dispersion is preferably not less than 1 wt% and not more than 10 wt%, and an alkaline concentration in the aqueous alkaline solution is preferably not less than 5 wt% and not more than 15 wt%.

The present invention relates to a method for purification using the adsorbent.

In addition, the present invention relates to a method for treatment using the adsorbent.

### EFFECT OF THE INVENTION

According to the present invention, the adsorbent having high adsorption capacity and high strength can be prepared by using porous cellulose beads obtained without using an auxiliary material which is highly toxic and corrosive and without a cumbersome step which is not suitable for an industrial production.

### BRIEF DESCRIPTION OF THE DRAWING

[Fig.1] Fig.1 is a SEM photograph of the surface of porous cellulose beads according to the present invention obtained in Example 1.
[Fig.2] Fig.2 is a SEM photograph of the surface of porous cellulose beads according to the present invention obtained in Example 2.
[Fig.3] Fig.3 is a SEM photograph of the surface of porous cellulose beads according to the present invention obtained in Example 3.
[Fig.4] Fig.4 is a SEM photograph of the surface of porous cellulose beads according to the present invention obtained in Example 4.
[Fig.5] Fig.5 is a SEM photograph of the surface of porous cellulose beads according to the present invention obtained in Example 5.
[Fig.6] Fig.6 is a SEM photograph of the surface of porous cellulose beads according to the present invention obtained in Example 6.
[Fig.7] Fig.7 is a SEM photograph of the surface of porous cellulose beads according to the present invention obtained in Example 7.

[Fig.8] Fig.8 is a SEM photograph of the surface of porous cellulose beads according to the present invention obtained in Example 8.

[Fig.9] Fig.9 is a SEM photograph of the surface of porous cellulose beads according to the present invention obtained in Example 9.

[Fig.10] Fig.10 is a SEM photograph of the surface of porous cellulose beads according to the present invention obtained in Example 10.

[Fig.11] Fig.11 is a SEM photograph of the surface of porous cellulose beads according to the present invention obtained in Example 11.

[Fig.12] Fig.12 is a SEM photograph of the surface of porous cellulose beads according to the present invention obtained in Example 12.

[Fig.13] Fig.13 is SEM photographs of the surface of porous cellulose beads according to the present invention obtained in Examples 13, 15 and 16.

[Fig.14] Fig.14 is SEM photographs of the surface of porous cellulose beads according to the present invention obtained in Examples 14, 17 and 18.

[Fig.15] Fig.15 is a SEM photograph of the surface of an adsorbent according to the present invention obtained in Reference Example 1.

[Fig.16] Fig.16 is a schematic perspective view of impeller used in Manufacture Example 1.

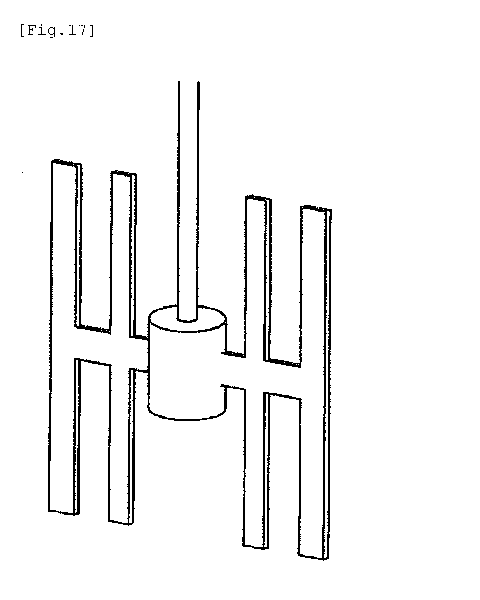

[Fig.17] Fig.17 is a schematic perspective view of impeller used in Manufacture Example 9.

MODE FOR CARRYING OUT THE INVENTION

The adsorbent according to the present invention is characterized by using porous cellulose beads obtained by mixing a cold alkaline aqueous solution and cellulose powder as a raw material to prepare a cellulose dispersion and bringing the cellulose dispersion into contact with a coagulating solvent.

When cellulose powder as a raw material having normal size is fed to a cold alkaline aqueous solution, dispersion in which the solution hardly turns into transparent as known well is obtained. However, porous cellulose beads can be prepared at more convenient and inexpensive even from such cellulose dispersion. In addition, obtained porous cellulose beads are excellent in strength.

The adsorbent of the present invention in which a ligand is bound to the porous cellulose beads is excellent in an adsorption capacity compared with an adsorbent obtained by using conventional porous cellulose beads. Even when cellulose is not dissolved in an alkaline aqueous solution, a special cluster is formed from water and an alkaline component at low temperature, cellulose is coordinated with the cluster to be swelled, and the cluster is absorbed in and replaced by a coagulating solvent; as a result, many pores are formed while coagulating the cellulose, to form a porous structure capable of utilizing as an adsorbent. Further, since formed pores are different from porous cellulose beads prepared by a conventional method, the adsorbent of the present invention is excellent in adsorptivity.

The adsorbent of the present invention is not particularly limited, as long as the adsorbent is used in applications capable of utilizing functions to adsorb or elute (release) a molecular to be targeted (subject matter). Preferably, the adsorbent can be used in affinity chromatography because the feature usable in a wide pH range is fully exhibited.

In order to use as the adsorbent for affinity chromatography, the affinity ligand for specifically binding the subject matter to be adsorbed can be introduced on porous cellulose beads.

The introduced amount of the affinity ligand in the adsorbent of the present invention is preferably not less than 1 mg and not more than 500 mg per 1 mL of the adsorbent. The introduced amount of the affinity ligand is preferably not less than 1 mg per 1 mL of the adsorbent in terms of increasing the amount of a target substance to be adsorbed, and is preferably not more than 500 mg in terms of limiting production cost. The introduced amount of the affinity ligand is more preferably not less than 2 mg and not more than 120 mg, further preferably not less than 3 mg and not more than 60 mg, particularly preferably not less than 4 mg and not more than 30 mg, and most preferably not less than 4 mg and not more than 15 mg, per 1 mL of the adsorbent. In the case where the affinity ligand is a non-oriented affinity ligand, the introduced amount of the affinity ligand in the adsorbent of the present invention is, for example, not less than 10 mg and not more than 80 mg, and preferably not less than 20 mg and not more than 50 mg per 1 mL of the adsorbent.

The introduced amount of the affinity ligand in the adsorbent of the present invention is preferably not less than 0.01 $\mu$mol and not more than 15 $\mu$mol per 1 mL of the adsorbent. The introduced amount of the affinity ligand is preferably not less than 0.01 $\mu$mol per 1 mL of the adsorbent in terms of increasing the amount of the target substance to be adsorbed, and is preferably not more than 15 $\mu$mol in terms of limiting production cost. The introduced amount of the affinity ligand is more preferably not less than 0.03 $\mu$mol and not more than 5 $\mu$mol, further preferably not less than 0.05 $\mu$mol and not more than 2 $\mu$mol, particularly preferably not less than 0.1 $\mu$mol and not more than 0.75 $\mu$mol, and most preferably not less than 0.1 $\mu$mol and not more than 0.5 $\mu$mol, per 1 mL of the adsorbent.

The introduced amount of the affinity ligand can be obtained by a method for measuring the absorbance from the affinity ligand in the supernatant of the reaction mixture after the immobilization reaction and BCA method (ANALYTICAL BIOCHEMISTRY 191, 343-346 (1990)) and the like. For example, in the case of an amino group-containing affinity ligand, it is possible to measure the introduced amount of the affinity ligand by subjecting the adsorbent to nitrogen content analysis.

An affinity ligand used in medical adsorbents for treatment, adsorbents for purifying antibody drugs and the like can include, for example, an antigen having high specificity to an antibody, a protein such as protein A, protein G and protein L and the variant thereof, or a peptide having activity of binding an antibody. In particular, attention has been focused on adsorbents obtained by immobilizing protein A as an affinity ligand on a base matrix as adsorbents capable of specifically adsorbing and eluting immunoglobulin (IgG) and the like. Attention has been focused on adsorbents obtained by immobilizing protein A as adsorbents for the treatment of rheumatism, hemophilia and dilated cardiomyopathy. In addition, adsorbents that enable the purification of antibodies such as IgG to be carried out on a large scale, at high speed and at low cost are needed in the field of antibody drug purification. From the perspective, the adsorbent of the present invention is preferably an adsorbent obtained by introducing protein A as an affinity ligand.

The protein A which can be used in the present invention is not particularly limited, and it is possible to use a natural product or a genetically modified product without limitation. In addition, a protein that contains a domain for binding an antibody or a variant thereof, a fusion protein or the like can be used as the protein A. In addition, it is possible to use protein A produced from a bacterial extract or culture supernatant by combining and/or repeating purification methods selected from among chromatography methods such as ion exchange chromatography, hydrophobic interaction chromatography, gel filtration chromatography and hydroxyapatite chromatography and methods such as molecular weight fractionation and fractional precipitation that use membrane separation technologies. In particular, protein A obtained using the methods disclosed in PCT Publication No. WO 2006/004067 or U.S. Patent No. US 5151350 can be preferably used.

Adsorbents are required to have a large adsorption capacity and various characteristics according to applications. For example, it is required that the subject substances are collected at high concentration in an application for purification. This is generally called as a collection step or an elution step, and in this step, it is required that a ligand easily releases the subject substances. In the case of using protein A as a ligand, the collection step indicates preferably a characteristic that an antibody is easily released, and protein A can be suitably used in the present invention. For example, protein A includes protein A described in WO 2011/118699. In addition, adsorbents are reused in many cases, and it is necessary that washing treatment or regeneration treatment is carried out for reuse. In purification of antibodies, since a solution containing urea, guanidine or the like is used, and the preparation of the solution is cumbersome, a method for washing and regenerating adsorbents with a sodium hydroxide aqueous solution has been a mainstream.

In the present invention, a ligand having an alkaline resistant can be suitably used. Also, protein A having an alkaline resistance can be preferably used. For example, the protein A having the alkaline resistance includes protein A described in WO2011/118699.

In addition, the protein A is preferably orientation-controlled protein A. Orientation-controlled protein A indicates protein A having a sequence capable of binding beads with a terminal part of protein A in the case of immobilizing protein A on cellulose beads.

A method for immobilizing a variety of affinity ligands such as protein A on porous beads can be selected from among a variety of immobilization methods, such as cyanogen bromide method, trichlorotriazine method, epoxy method and tresyl chloride method. In particular, from an industrial perspective, it is preferable for immobilization to use the reaction between a formyl group on porous beads and an amino group on an affinity ligand in terms of safety and the ease of the immobilization reaction and the availability of proteins or peptides produced by a relatively simple method (for example, WO2010/064437). From these perspectives, it is desirable that the orientation-controlled protein A has an amino acid sequence in which all of Lys (lysine residue) of amino acid sequences from any of E domain, D domain, A domain, B domain, and C domain of protein A are substituted with amino acid variations, and in which Lys is provided with a terminal.

In addition, since there are cases where the introduction amount of the protein A on beads is decreased, an amount of an active group of beads for immobilizing a ligand is preferably not less than 1 $\mu$mol per 1 mL of beads. When the amount of the active group is much large, an amount of an active group is preferably not more than 500 $\mu$mol per 1 mL of beads because treatments of inactivating the active group after the immobilization of the ligand become cumbersome. The amount of the active group is more preferably not less than 5 $\mu$mol and not more than 250 $\mu$mol, even preferably not less than 10 $\mu$mol and not more than 125 $\mu$mol, especially preferably not less than 20 $\mu$mol and not more than 60 $\mu$mol, and most preferably not less than 30 $\mu$mol and not more than 50 $\mu$mol per 1 mL of beads.

It is preferable that the active group on beads is a formyl group from the viewpoint of ease of binding a ligand, or simple provision of many active groups by binding a compound having active groups.

The adsorption capacity of the subject substances of adsorbents of the present invention can be obtained by a method for measuring 5 % dynamic binding capacity (DBC) in a state that adsorbents are packed in a column.

In the case where adsorption treatment is carried out for residence time (hereinafter, referred to as RT) of 3 minutes, an adsorption capacity of the subject substance is preferably not less than 1 g per 1 L of adsorbents. It is preferable that the adsorption capacity of the subject substance is not less than 1 g per 1 L of adsorbents due to effective purification. In addition, it is preferable that the adsorption capacity of the subject substance is not more than 200 g per 1 L of adsorbents because adsorbed subject substances are easily eluted from adsorbents. The adsorption capacity of the subject substance is more preferably not less than 10 g and not more than 150 g, even preferably not less than 30 g and not more than 100 g, especially preferably not less than 50 g and not more than 90 g, and most preferably not less than 60 g and not more than 80 g per 1 L of adsorbents.

In the case where adsorption treatment is carried out for residence time (RT) of 6 minutes, the adsorption capacity of the subject substance is preferably not less than 20 g and not more than 200 g, more preferably not less than 40 g and not more than 150 g, especially preferably not less than 60 g and not more than 100 g, and most preferably not less than 70 g and not more than 90 g per 1 L of adsorbents.

Dextran sulfate can be preferably used as other ligand of the adsorbent of the present invention. In the case of using dextran sulfate, an adsorbent suitable for each of various ionic exchange chromatography can be obtained. In addition, dextran sulfate is suitable as a ligand used in adsorbents for adsorbing LDL cholesterol, and an adsorbent immobilized with dextran sulfate as a ligand on a carrier can be used as a medical adsorbent for adsorbing and removing LDL cholesterol.

An adsorption capacity of LDL cholesterol is preferably not less than 1 g and not more than 50 g per 1 L of adsorbents. In the case of not less than 1 g, a suitable treatment can be carried out when an adsorbent immobilized with dextran sulfate on a carrier is used for the medical adsorbent. In the case of not more than 50 g, side-effect from rapid decrease in LDL cholesterol can be suppressed. An adsorption capacity of LDL cholesterol is preferably not less than 2 g and not more than 20 g, especially preferably not less than 4 g and not more than 15 g, and most preferably not less than 6 g and not more than 10 g per 1 L of adsorbents.

Hereinafter, each step of a method of preparing porous cellulose beads used in the present invention is described.

(1) Step for preparing a cellulose dispersion

In a step of preparing a porous cellulose bead used in the present invention, a cold alkaline aqueous solution and cellulose powder as a raw material are mixed. A reaction in which cellulose powder as a raw material is solvated by a cold alkaline aqueous solution is an exothermic reaction; therefore, when cellulose is added to an alkaline aqueous solution having high temperature, a dispersion which is homogenous and colorless cannot be obtained. Therefore, the low temperature is maintained at the time of mixing cellulose with an alkaline aqueous solution.

In the present invention, the term "low temperature" means a temperature which is less than an ambient temperature. As long as the temperature is less than an ambient temperature, there is no big problem. A temperature of the alkaline aqueous solution is not less than -20°C is preferred, since a temperature control system can be simple and running cost of a temperature control system can be lowered. In addition, a temperature of not more than 10 °C is preferred, coloration of the cellulose dispersion is decreased and dispersibility and swellablility of cellulose become higher. The temperature of the alkaline aqueous solution is more preferably not less than -10°C and not more than 20°C. When the temperature is not less than -10°C, it can be prevented to freeze an alkaline aqueous solution. On the other hand, when the temperature is not more than 20°C, the cellulose dispersion can be effectively prepared and coloration of the cellulose dispersion can be decreased. The temperature of the alkaline aqueous solution is more preferably not less than -5°C, even more preferably not less than -2°C, and particularly preferably not less than -1°C. The temperature of the alkaline aqueous solution is more preferably not more than 15°C, even more preferably not more than 9°C, even more preferably not more than 5°C, even more preferably not more than 4°C, and even more preferably not more than 1°C. In addition, the temperature of not more than 9°C is preferred, since sphericity of the obtained porous cellulose beads can become higher.

An alkali to be used is not particularly limited as long as an aqueous solution shows alkalinity. As such an alkali, lithium hydroxide, sodium hydroxide and potassium hydroxide are preferred from the viewpoint of ready availability, and sodium hydroxide is most preferred from the viewpoint of price and product safety.

An alkali concentration in the alkaline aqueous solution is not particularly limited, and is preferably not less than 3 wt% and not more than 20 wt%. When the concentration of the alkaline is included in the range, dispersibility and swellability of cellulose in the alkaline aqueous solution become high. The alkaline concentration in the alkaline aqueous solution is more preferably not less than 5 wt% and not more than 15 wt%, even more preferably not less than 7 wt% and not more than 10 wt%, and most preferably not less than 8 wt% and not more than 10 wt%.

The kind of the cellulose powder as a raw material to be used is not particularly limited. For example, in the present invention, it is not necessary to use substituted cellulose such as cellulose substituted with a substituent for improving solubility, and common unsubstituted cellulose powder can be used as a raw material, since cellulose is not needed to be dissolved.

A molecular weight of cellulose as a raw material is not particularly limited, but the degree of polymerization of cellulose is preferably not more than 1000. When the degree of polymerization is not more than 1000, dispersibility and swellability of cellulose in the alkaline aqueous solution become higher. In addition, the degree of polymerization of not less than 10 is preferred, since mechanical strength of the obtained porous cellulose beads becomes higher. The degree of polymerization is more preferably not less than 50 and not more than 500, even more preferably not less than 100 and not more than 400, particularly preferably not less than 200 and not more than 350, and most preferably not less than 250 and not more than 350.

A median particle diameter of the cellulose powder as a raw material is preferably not less than 10 $\mu$m and not more than 500 $\mu$m. It is unnecessary to pulverize cellulose powder as a raw material with a special method for improving solubility since the present invention does not adopt means of dissolving cellulose. In addition, when cellulose powder as a raw material is excessively pulverized, the overall productivity is decreased. In other words, cellulose is conventionally pulverized finely by a special method such as blasting treatment and wet grinding for dissolving cellulose in a cold alkaline aqueous solution; however, the production cost is raised due to such a treatment. Therefore, the median particle diameter of cellulose powder as a raw material is preferably not less than 10 $\mu$m. In addition, when the median particle diameter is not less than 10 $\mu$m, clumping is hardly caused in the cellulose dispersion. On the other hand, when cellulose powder as a raw material of which median particle diameter is too large is used, a stable dispersion cannot be obtained and eventually porous cellulose beads may not be possibly produced in an effective way. Therefore, the median particle diameter is preferably not more than 500 $\mu$m. The median particle diameter is more preferably not less than 15 $\mu$m, even more preferably not less than 20 $\mu$m, particularly preferably not less than 45 $\mu$m, and more preferably not more than 200 $\mu$m. It is a preferred condition that the median particle diameter of cellulose powder as a raw material is, for example, not less than 5 $\mu$m and not more than 50 $\mu$m, and especially preferably not less than 10 $\mu$m and not more than 30 $\mu$m.

In addition, a dissolving pulp is exemplified as cellulose powder as a raw material of which solubility is improved. The dissolving pulp may be actually used as a raw material for producing the cellulose dispersion used in the present invention; however, it is well-known that a dissolving pulp may be produced commonly by a method of which environmental load is heavy. Also, the present inventors know that nowadays the dissolving pulp is very difficult to be obtained as a raw material for producing porous cellulose beads probably due to a structural problem of cellulose industries. According to the present invention, porous cellulose beads can be effectively produced without using the dissolving pulp. Therefore, generally and easily available cellulose is preferably used in the present invention for decreasing a total production cost and improving productivity in order to obtain a porous cellulose bead used in the present invention.

A condition to mix an alkaline aqueous solution with cellulose powder as a raw material is not particularly limited. For example, cellulose powder as a raw material may be added into an alkaline aqueous solution, and an alkaline aqueous solution may be added to cellulose powder as a raw material. It is preferred that an alkaline aqueous solution is preliminarily cooled down to a low temperature and then cellulose powder as a raw material is added to the cooled alkaline aqueous solution.

Cellulose powder as a raw material may be suspended in water before mixing with an alkaline aqueous solution. As a result, clumping of cellulose can be prevented, and the time required for preparing the cellulose dispersion can be reduced, and the cellulose dispersion which is more homogeneous can be readily obtained. A ratio of cellulose in the suspension can be arbitrarily controlled, and is exemplified by not less than 1 wt% and not more than 40 wt%.

It is also preferred that cellulose powder as a raw material or a suspension of cellulose powder as a raw material is cooled down to a low temperature similarly to an alkaline aqueous solution before mixing with an alkaline aqueous solution. In such a case, a temperature of cellulose powder as a raw material or a suspension of cellulose powder as a raw material may not be the same as a temperature of an alkaline aqueous solution.

It is preferred to stir an alkaline aqueous solution to which cellulose powder as a raw material or a suspension of cellulose powder as a raw material is added, or a suspension of cellulose powder as a raw material to which an alkaline aqueous solution is added. A value of Pv, which represents a stirring power in such a case, is preferably not less than 0.01 kW/m$^3$ and not more than 100 kW/m$^3$. When the stirring power is not less than 0.01 kW/m$^3$, both solutions can be efficiently mixed. In addition, when the stirring power is too high, it may be possibly difficult to be mixed in some cases. Therefore, the stirring power is preferably not more than 100 kW/m$^3$.

Also, the present inventors surprisingly found that when a cellulose suspension obtained by dispersing cellulose powder as a raw material in water is cooled down to a low temperature and then an alkaline aqueous solution is added to the stirred suspension, a homogeneous cellulose dispersion can be instantaneously prepared. Especially, such a method is preferably used. In such a case, it is more preferred that a temperature of the alkaline aqueous solution to be added is low. It is also preferred that the cellulose dispersion is maintained at a low temperature during both of preparation and preservation. The temperature can be the same as the above-described temperature of an alkaline aqueous solution.

A cellulose concentration in the cellulose dispersion is preferably not less than 1 wt% and not more than 10 wt%. When the concentration is not less than 1 wt%, mechanical strength of the obtained porous cellulose beads becomes higher. The concentration of not more than 10 wt% is preferred, since viscosity of the cellulose dispersion becomes lower and an amount of cellulose which is not be dispersed or swelled is reduced. The cellulose concentration in the cellulose dispersion is more preferably not less than 3 wt% and not more than 10 wt%, even more preferably not less than 4 wt% and not more than 8 wt%, particularly preferably not less than 5 wt% and not more than 7 wt%, and most preferably not less than 5 wt% and not more than 6 wt%. When the concentration of cellulose in the cellulose dispersion is calculated, cellulose which is not dispersed nor swelled and which is not homogeneously dispersed is not counted.

(2) Step for preparing an emulsion

The cellulose dispersion may be dispersed in a dispersive medium to prepare an emulsion, and then the emulsion may be brought into contact with a coagulating solvent. A preparation of such an emulsion is optional. However, when the step for preparing an emulsion is undergone, porous cellulose beads may be readily obtained from the cellulose dispersion of which cellulose amount to be contained is relatively large. In addition, porous cellulose beads of which mechanical strength is high may be readily obtained.

A dispersion medium is not particularly limited, and a dispersion medium of which compatibility with the cellulose dispersion is low is preferably used. For example, an edible oil such as medium chain fatty acid triglyceride (MCT); a natural oil such as palm oil, coconut oil and squalene; a higher alcohol such as isostearyl alcohol and oleyl alcohol; a higher ester such as 2-octyldodecanol; a lipophilic organic solvent such as dichlorobenzene can be used. In addition, an appropriate amount of surfactant such as a sorbitan fatty acid ester such as sorbitan laurate, sorbitan stearate, sorbitan oleate and sorbitan trioleate may be added to a dispersion medium.

An amount of a dispersion medium may be adjusted to sufficiently disperse droplets of a cellulose dispersion. For example, a ratio of the dispersion medium may be not less than one time by mass relative to a cellulose dispersion. On the other hand, when an amount of a dispersion medium is too much, an amount of waste liquid may be excessively increased. Therefore, the ratio of the dispersion medium is preferably not more than 10 times by mass. The ratio of the dispersion medium is more preferably not less than 2 times by mass, even more preferably not less than 4 times by mass, and more preferably not more than 8 times by mass, even more preferably not more than 7 times by mass, and particularly preferably not more than 6 times by mass.

It is preferred to adjust a temperature during the dispersion as similar to a temperature of the cellulose dispersion. In other words, it is preferred that a temperature of a dispersion medium, a temperature when a dispersion medium and a cellulose dispersion are mixed and a temperature when a cellulose dispersion is dispersed in a dispersion medium are adjusted to be cool similarly to a temperature of the alkaline aqueous solution.

It is usually preferred that when an emulsion is prepared, a cellulose dispersion is added to a stirred dispersion medium. A Pv value of a stirring power during preparation of the emulsion is preferably not less than $0.1$ kW/m$^3$ and not more than $12$ kW/m$^3$. When the stirring power is not less than $0.1$ kW/m$^3$, good sphericity and porous property may be readily achieved. In addition, when the stirring power is too high, flowability of the emulsion may be possibly difficult to be stable; therefore, the stirring power is preferably not more than $12$ kW/m$^3$. The stirring power is more preferably not less than $1.1$ kW/m$^3$, even more preferably not less than $3.1$ kW/m$^3$, and particularly preferably not less than $5.5$ kW/m$^3$.

(3) Step for coagulation

Next, the cellulose dispersion is brought into contact with a coagulating solvent to form porous cellulose.

A coagulating solvent used in the present step is not particularly limited as long as when the cellulose dispersion is brought into contact with the solvent, cellulose beads can be obtained. Specifically, water and an alcohol solvent are suitably used, since these solvents have high affinity with the alkaline aqueous solution, which is a good solvent of the cellulose dispersion. In particular, an alcohol solvent is preferred, since when the solvent is used, a pore size of cellulose beads can be micrified in comparison with the case of using water. In addition, an alcohol solvent is preferred, since when the solvent is used, sphericity is improved. A mixed solvent of water and an alcohol is more preferred, since when the mixed solvent is used, a pore size of cellulose beads can be arbitrarily adjusted by changing a mixing ratio.

An alcohol used in the present invention is not particularly limited, and alcohol of which carbon number is not more than 6 is preferred since such an alcohol has high affinity with the alkaline aqueous solution. An alcohol of which carbon number is not more than 4 is more preferred, and methanol is most preferred. A coagulating solvent may be an alcohol aqueous solution.

It is also preferred that the coagulating solvent used in the present invention is acidified. When the coagulating solvent is acidified, the alkaline aqueous solution can be preferably neutralized. When the alkaline aqueous solution is rapidly neutralized, a chemical damage of the obtained cellulose beads can be reduced. In addition, the present inventors surprisingly found that when the coagulating solvent is acidified, pore size distribution of the obtained porous beads becomes narrower. Therefore, when such a pore diameter distribution property is desired, it is particularly preferred that the coagulating solvent is acidified. A reagent for acidification is not particularly limited, and an inorganic acid such as sulfuric acid and hydrochloric acid, an organic acid such as acetic acid, citric acid and tartaric acid, an acid having buffering action such as phosphate and carbonate, and the like can be widely used. The pH of the coagulating solvent may be adjusted to less than 7.0 for acidifying the coagulating solvent. The pH is preferably not more than 5.0, more preferably not more than 4.0, even more preferably not more than 3.0, and particularly preferably not more than 2.0. The lower limit of the pH is not particularly limited, and the pH is preferably not less than 0.0.

An amount of the coagulating solvent to be used is not particularly limited, and may be appropriately adjusted. For example, the amount may be adjusted to not less than 0.001 times by volume and not more than 100 times by volume relative to the cellulose dispersion. The amount may be adjusted to not less than 0.01 times by volume and not more than 10 times by volume relative to the emulsion. When the amount is within the above-described range, porous cellulose beads can be efficiently produced and desirable pores and surface pores can be efficiently formed. An amount of the coagulating solvent to be used relative to the emulsion is more preferably not less than 0.025 times by volume, even more preferably not less than 0.05 times by volume, particularly preferably not less than 0.07 times by volume, and more preferably not more than 0.4 times by volume, even more preferably not more than 0.2 times by volume, and particularly preferably not more than 0.15 times by volume. The above amount to be used is considerably small in comparison with a usual amount of a coagulating solvent. However, even when an amount of the coagulating solvent to be used is decreased, porous cellulose beads can be efficiently obtained according to the present invention.

A temperature of the coagulating solvent is not particularly limited, and the temperature of the coagulating solvent is preferably a temperature such that the cellulose dispersion is not frozen, since when the cellulose dispersion is frozen in the coagulating solvent and then melted, cellulose beads may become deformed or cellulose may be crushed. In addition, a temperature of the coagulating solvent is preferably not less than a temperature of the cellulose dispersion. In general, a temperature of the coagulating solvent is adjusted to less than a temperature of the cellulose dispersion in order to increase a coagulating speed. On the other hand, the present inventors surprisingly found that coagulation progresses at a faster rate by adjusting a temperature of the coagulating solvent to not less than a temperature of the cellulose dispersion. A specific temperature of the coagulating solvent is not particularly limited, and the temperature of the coagulating solvent is preferably not less than 0°C and not more than 150°C, more preferably not less than 25°C and not more than 100°C, and even more preferably not less than 45°C and not more than 80°C. However, it is preferred to appropriately adjust the temperature in consideration of a boiling temperature of the coagulating solvent or the like. In the present invention, a temperature of the cellulose dispersion means a temperature of the emulsion when the emulsion is used.

It is a preferred condition that the temperature of the coagulating solvent is, for example, not less than 0 °C and not more than 10 °C, and particularly preferably not less than 2 °C and not more than 6 °C.

(4) Step for crosslinking

Strength of porous cellulose beads obtained by the above-described method can be further improved by using a crosslinking agent. Crosslinked porous cellulose beads are especially excellent in strength; therefore, such porous cellulose beads withstand under high linear velocity and high pressure. The present step may be optionally carried out.

A method for crosslinking is not particularly limited, and a publicly-known method may be applied. A crosslinking agent and crosslinking reaction condition are not also particularly limited, and publicly-known art may be applied. A crosslinking agent is exemplified by a halohydrin such as epichlorohydrin, epibromohydrin and dichlorohydrin; a bisfunctional bisepoxide (bisoxirane); and polyfunctionalpolyepoxide (polyoxirane). In particular, a method described in JP 2008-279366 is preferably applied. The present inventors developed a method described in JP 2008-279366 and found that strength of porous cellulose beads can be further improved by fractionally adding the alkaline aqueous solution for accelerating a crosslinking reaction. Such a crosslinking method can be applied to the present invention most preferably. The content of the above publication is incorporated by reference.

Stirring operation is preferably carried out in the step for preparing the cellulose dispersion, the step for preparing the emulsion and the step for coagulating. A stirring blade, not particularly limited, includes a paddle blade and a turbine blade. In particular, a pitched paddle blade, a rushton turbine blade and the like are preferably used.

The porous cellulose beads obtained as shown in the above preferably have a minute structure to exhibit excellent binding property to IgG in the case where an affinity ligand such as protein A is immobilized. In the porous cellulose beads having high binding property to IgG, specific surface area of the porous cellulose beads to which IgG is accessible is, for example, not less than $1 \times 10^7$ and not more than $30 \times 10^7$ $m^2/m^3$, preferably not less than $3 \times 10^7$ and not more than $20 \times 10^7$ $m^2/m^3$, and more preferably not less than $5 \times 10^7$ and not more than $15 \times 10^7$ $m^2/m^3$. In addition, in a porous cellulose beads having high binding property to IgG, a saturated adsorption capacity for IgG in theory is, for example, not less than 50 g/L and not more than 200 g/L, preferably not less than 60 g/L and not more than 150 g/L, and more preferably not less than 70 g/L and not more than 120 g/L.

By use of the adsorbent of the present invention, effective purification and treatment can be carried out.

The present application claims the benefit of the priority date of Japanese patent application No. 2012-198352 filed on September 10, 2012, and all of the contents of the Japanese patent application No. 2012-198352 filed on September 10, 2012, are incorporated by reference.

EXAMPLES

Hereinafter, the present invention is described with Examples; however, the present invention is not limited to the Examples. First, test methods of physical properties of a porous cellulose beads or an adsorbent prepared is explained.
In Examples as set forth below, when a concrete amount corresponding to 1 part by weight is 1 kg, a concrete amount corresponding to 1 part by volume is 1 L.

Test Example 1:

Observation of surface of beads by SEM

The porous cellulose beads or adsorbents obtained in each of Manufacture Examples and Reference Examples were washed with 30% ethanol in a 5 times more volume than that of the beads or the adsorbents, and the liquid part contained in the porous cellulose beads was substituted by 30% ethanol. Next, the porous cellulose beads were similarly treated to substitute the liquid part thereof with ethanol by using 50% ethanol, 70% ethanol, 90% ethanol, special grade ethanol, special grade ethanol and special grade ethanol in order. Further, the porous cellulose beads were similarly treated using a mixed solvent of t-butyl alcohol / ethanol = 3 / 7. Then, the porous cellulose beads were similarly treated to substitute the liquid part thereof with t-butyl alcohol by using mixed solvents of t-butyl alcohol / ethanol = 5/5, 7/7, 9/1, 10/0, 10 / 0 and 10/0 in this order. Then, the porous cellulose beads were freeze-dried. The freeze-dried porous cellulose beads were subjected to vapor deposition process to photograph SEM images.

Test Example 2:

Measurement of exclusion limit molecular weight and maximum pore diameter

(1) Packing in column

The porous cellulose beads or adsorbents were dispersed in RO water, and the mixture was degassed for one hour. A column (Tricorn 10/300, manufactured by GE Healthcare Japan Corporation) was packed with the degassed porous cellulose beads or adsorbents at a linear velocity of 105 cm/h. Next, an eluent (129 mL) of which pH was 7.5 was passed through the column at a linear velocity of 26 cm/h.

(2) Addition of marker

The following markers were used.

- Blue Dextran 2000, manufactured by Pharmacia FIne Chemicals Co. , Ltd.
- Low Density Lipoprotein, manufactured by SIGMA-Aldrich Co., Ltd., MW 3,000,000
- Thyroglobulin, manufactured by SIGMA-Aldrich Co., Ltd., MW 660,000
- Ferritin, manufactured by SIGMA-Aldrich Co., Ltd., MW 440,000
- Aldolase, manufactured by SIGMA-Aldrich Co., Ltd., MW 158,000
- IgG derived from human, manufactured by SIGMA-Aldrich Co., Ltd., MW 115,000 (not used in Reference Example 1)
- Bovine Serum Albumin, manufactured by Wako Pure Chemical Industries, Ltd., MW 67,000
- Cytochrome C, manufactured by Wako Pure Chemical Industries, Ltd., MW 12,400
- Bacitracin, manufactured by Wako Pure Chemical Industries, Ltd., MW 1,400

The above markers were diluted with buffer of pH 7.5 to adjust the concentration to be 5 mg/mL. While the above eluent was passed through the column at a linear velocity of 26 cm/h, 12 $\mu$L of each diluted solution was injected. The concentrations of the markers were finely tuned as necessary.

(3) Measurement

As measurement device, DGU-20A3, SCL-10A, SPD-10A, LC-10AD, SIL-20AC and CTO-10AC, which were respectively manufactured by SHIMADZU Corporation, were used, and LCSolution was used as software for measurement. For measuring an amount of liquid, 50 mL graduated cylinder was used.

At the same time as the injection of the marker, observation by UV monitor and measurement of eluent amount were started, and

1) an eluent amount corresponding to the first peak of Blue Dextran was measured as $V_0$ mL;
2) eluent amounts corresponding to the peaks of each markers were measured as $V_R$ mL;
3) a total volume of porous cellulose beads or adsorbents in the column was regarded as $V_t$ mL.

(4) Calculation

The value of $K_{av}$: gel phase distribution coefficients of each markers was calculated in accordance with the following formula.

$$K_{av} = (V_R - V_0) / (V_t - V_0)$$

(5) Calculation of exclusion limit molecular weight and maximum pore diameter

The value of $K_{av}$ and logarithm of molecular weight of each markers were plotted, and the slope and y-intercept of the following formula were obtained from the part which exhibited linearity.

$$K_{av} = k \times L_n \text{ (molecular weight)} + b$$

Then, the molecular weight when $K_{av}$ is 0, i. e. exclusion limit molecular weight, was obtained from the above slope and intercept. Next, the exclusion limit molecular weight was substituted in the following correlation formula of diameter and molecular weight of globular protein in a neutral buffer solution, and the value was obtained as the maximum diameter of the pores of the sample particle.

$$\text{Diameter of globular protein in a neutral buffer solution (Å)}$$
$$= 2.523 \times \text{(molecular weight)}^{0.3267}$$

Test Example 3:

Calculation of average pore diameter

The molecular weight corresponding to the value of the maximum $K_{av}$ / 2 in the part which exhibited linearity in the Test Example 2(5) was substituted in the above-described correlation formula of diameter and molecular weight of globular protein in a neutral buffer solution, and the value was obtained as an average pore diameter of the porous cellulose beads or adsorbents.

When $K_{av}$ of adsorbent for a target substance to be adsorbed was measured in the Test Example 2 and Test Example 3, the target substance could be adsorbed and an accurate measurement might be impossible. Therefore, the value of $K_{av}$ of adsorbent for the target substance to be adsorbed was obtained by measuring the values of $K_{av}$ of two or more proteins which had molecular weight close to the molecular weight of the target substance and then calculating from the measured data. For example, when the target substance to be adsorbed is IgG, the value of $K_{av}$ was obtained from the data of ferritin and albumin.

Test Example 4:

Calculation of specific surface area to which IgG is accessible (hereinafter referred to as specific surface area in some cases)

Specific surface area of absorbents to which IgG is accessible was calculated, assuming that a minute structure of porous cellulose beads was a cylindrical pore corresponding to a diameter of each of marker molecules. Concretely, a linear line was obtained by utilizing a formula having linearity obtained in the above Test Example 2 (5), and a correlated formula between a diameter and a molecular weight of a globular protein in a neutral buffer solution, and plotting $K_{av}$

and pore sizes from exclusion limit molecular weight to a molecular weight of IgG (146,000 of molecular weight in the present test example). The obtained linear line was arbitrarily divided in an interval, a wall area of cylindrical pores for each intervals was obtained from each interval $K_{av}$ (which was regarded to be a volume of cylindrical pores of each intervals), and values of each of wall areas were accumulated to a point to corresponding to IgG, to obtain specific surface area of porous cellulose beads to which IgG is accessible.

Test Example 5

Calculation of saturated adsorption capacity for IgG in theory

A saturated adsorption capacity for IgG in theory was calculated from specific surface area of porous cellulose beads to which IgG is accessible obtained in Test Example 4. Concretely, the number of IgG per a unit volume in porous cellulose beads was obtained with the following formula.

```
Number of IgG per unit volume = specific surface area of porous cellulose beads to which IgG is accessible ÷ occupation area of

IgG
```

A weight of IgG per a unit volume (volume of precipitated beads) was obtained from the number of IgG calculated, to obtain a saturated adsorption capacity for IgG in theory (assuming that a ratio of packing of beads at the time of precipitation was 60 %).
Here, occupation area of IgG was obtained from a correlation formula of a diameter and a molecular weight of globular proteins in a neutral buffer solution.

Test Example 6:

Measurement of median particle diameter

Particle size distribution of the porous cellulose beads on the basis of volume was measured using a laser diffraction / scattering type particle size distribution measuring apparatus (LA-950, manufactured by HORIBA Ltd.), to obtain median particle diameter of porous cellulose beads.

Test Example 7:

Evaluation of strength

AKTAexplorer 10S (manufactured by GE Healthcare Bio-Sciences Corp.) was used, and 22 $\mu$m of mesh was attached to a column having a diameter of 0.5 cm and a height of 15 cm. 3 mL of the porous cellulose beads or adsorbents were packed into the column, and 20% ethanol aqueous solution (prepared from ethanol manufactured by Wako Pure Chemical Industries, Ltd. and distilled water) was passed at a linear velocity of 450 cm/h for 1 hour. Next, a phosphate buffer of pH 7.4 (manufactured by SIGMA-Aldrich Co. Ltd) was passed through the column at various linear velocities to specify the linear velocity at which critical compression was observed for strength evaluation.

Test Example 8

Measurement of dynamic binding capacity (DBC) for RT of 3 minutes

(1) Preparation of solution

The following solutions were prepared.
Solution A: phosphate buffer of pH 7.4 (manufactured by SIGMA-Aldrich Co. Ltd.)
Solution B: 35 mM sodium acetate of pH 3.5 (prepared with acetate manufactured by NACALAI TESQUE, INC., sodium acetate, and RO water)
Solution C: 1 M acetate (prepared with acetate manufactured by NACALAI TESQUE, INC., and RO water)
Solution D: 1 mg/mL of human polyclonal IgG solution (prepared with 1500 mg/10 mL gamma globulin nichiyaku manufactured by NIHON PHARMACEUTICAL CO., LTD, and A solution)
Solution E: 6 M urea (prepared with urea manufactured by KANTO CHEMICAL CO.INC. and RO water)
Each of solutions was defoamed prior to use.

(2) Packing, preparation

AKTAexplorer 100 (manufactured by GE Healthcare Bio-Sciences Corp.) was used as a device for column chromatography, and 22 μm of mesh was attached to a column having a diameter of 0.5 cm and a height of 15 cm. 3 mL of the porous cellulose beads or adsorbents were packed into the column, and 20% ethanol aqueous solution (prepared from ethanol manufactured by Wako Pure Chemical Industries, Ltd. and RO water) was passed at a linear velocity of 450 cm/h for 1 hour. Then, 15 ml of a collective tube was set to a fraction collector. A neutralizing solution was previously charged into the collective tube for the elution solution.

(3) Purification of IgG

9 mL of solution A was passed through the column at a linear velocity of 300 cm/h, and solution D was passed through the column at a linear velocity of 300 cm/h with monitoring by UV until 10 % of IgG was broken though. A loading amount of IgG at the time of 5% breaking through was regarded as 5 % DBC for RT of 3 minutes. Then, 30 mL of solution A was passed through the column at a linear velocity of 300 cm/h, and 30 mL of solution B was passed through the column at a linear velocity of 300 cm/h to elute IgG. Next, 9 mL of solution C was passed through the column at a linear velocity of 300 cm/h, and 9 mL of solution E was passed through the column at a linear velocity of 300 cm/h to carry out a regeneration treatment.

Test Example 9

Measurement of dynamic binding capacity for RT of 6 minutes

Dynamic binding capacity was obtained by changing a linear velocity of Test Example 8 (3) to a linear velocity of 150 cm/h.

Test Example 10

Measurement of dynamic binding capacity for RT of 9 minutes

Dynamic binding capacity was obtained by changing a linear velocity of Test Example 8 (3) to a linear velocity of 100 cm/h.

Test Example 11

Quantitative Evaluation of epoxy group

Epoxidized porous cellulose beads were subjected to suction filtration (suction dry) on glass filter (3G-2 manufactured by TOP) for 15 minutes, 1.5 g of porous cellulose beads subjected to suction dry was weighed in a screw tube (manufactured by Maruemu Corporation), and 4.5 mL of 1.3 M of sodium thiosulfate solution (prepared with sodium thiosulfate manufactured by Wako Pure Chemical Industries, Ltd. and RO water) was added thereto. The mixture was heated at 45 °C for 30 minutes, RO water was added such that a volume of the mixture was adjusted to 50 mL. The mixture was transferred to 100 mL of beaker made of glass. A few drops of 1% of phenolphthalein solution (prepared with phenolphthalein manufactured by Wako Pure Chemical Industries, Ltd. and ethanol) was added thereto. Titration by using 0.01 N hydrochloric acid (Wako Pure Chemical Industries, Ltd., grade for volume analysis) was carried out to obtain content of an epoxy group.

Test Example 12

Quantitative evaluation of introduced amount of dextran sulfate

Introduced amount of dextran sulfate was measured by utilizing the affinity between dextran sulfate and toluidine blue. That is, 120 mL of basic blue 17 (manufactured by TOKYO CHEMICAL INDUSTRY CO., LTD) adjusted to about 90 mg/L was added to 1 mL of adsorbents, a mixture was stirred for 10 minutes, and was stood for 60 minutes. Absorbance of basic blue of a supernatant was determined at 630 nm, to obtain an introduced amount of dextran sulfate from decreasing values thereof.

Manufacture Example 1

(1) Preparation of alkaline aqueous solution A

Sodium hydroxide manufactured by Wako Pure Chemical Industries, Ltd. and distilled water were used to prepare 33 wt% sodium hydroxide aqueous solution, and the temperature of the solution was adjusted to 4°C.

(2) Preparation of cellulose dispersion A

9.2 parts by weight of Pharmacopeia Cellulose PH-F20JP (manufactured by Asahi Kasei Chemicals Corporation (median particle diameter: 21 $\mu$m)) and 104 parts by weight of distilled water were mixed, and a temperature of a mixture was adjusted at 4 °C with stirring. Next, 40 parts by weight of the alkaline aqueous solution A adjusted at 4 °C with maintaining a preset temperature and stirring was fed thereto, to stir a mixture for 30 minutes.

(3) Preparation of porous cellulose beads

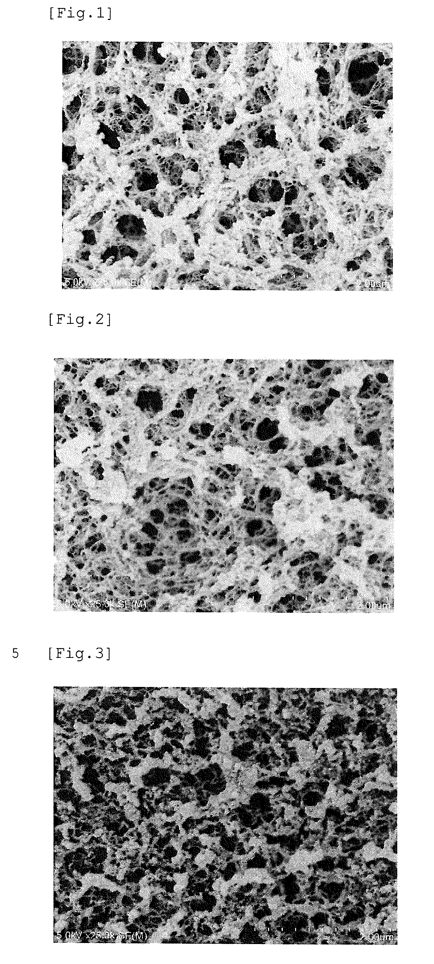

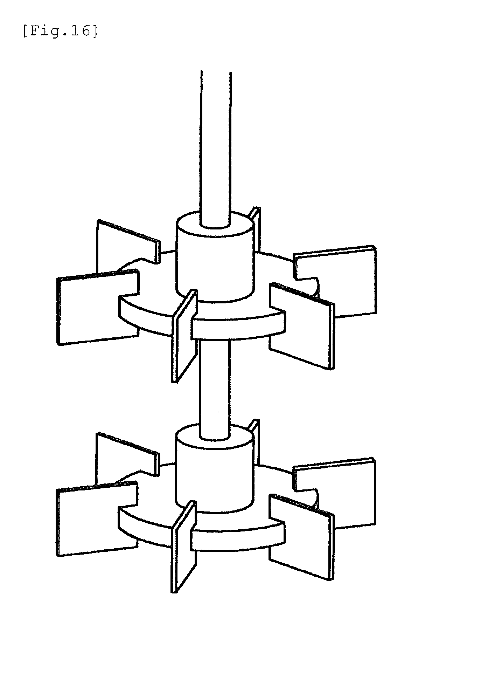

154 parts by weight of the cellulose dispersion A adjusted at 4 °C, 776 parts by weight of orthodichlorobenzene adjusted at 4 °C, 7.8 parts by weight of sorbitan monooleate adjusted at 4 °C (one corresponding to span 80) were mixed to obtain a mixture, the mixture was stirred at 4 °C for 30 minutes under condition of 300 rpm (Pv value: 0.2 kW/m$^3$) in a separable flask equipped with 2 pieces of rushton turbine paddles (see Fig. 16. similarly below), to obtain an emulsion. As a coagulate solvent, 57 parts by weight of methanol adjusted at 4 °C was added thereto with maintaining a preset temperature and stirring. The time needed to add the coagulate solvent was 2 seconds. A mixture was stirred for 20 minutes with maintaining the number of stirring and a preset temperature. After suction filtration was carried out, washing was carried out using 240 parts by weight of ethanol and subsequently 500 parts by weight of water to obtain porous cellulose beads. As shown in Fig. 1, it was confirmed that good pores were formed on the surface of beads. Obtained porous cellulose beads were subjected to wet classification by using sieves of 38 $\mu$m and 90 $\mu$m.

(4) Crosslinking - Method A, for which JP 2008-279366 A was referred

Distilled water was added to 11 parts by volume of the above porous cellulose beads such that the total volume became 16.5 parts by volume. The slurry was transferred to a reaction vessel. To the reaction vessel, 3.86 parts by volume of 4N NaOH aqueous solution which was prepared from NaOH manufactured by NACALAI TESQUE, INC. and distilled water was added. The mixture was heated up to 40°C. To the mixture, 1.77 parts by weight of a crosslinking agent (DENACOL EX-314, manufactured by Nagase ChemteX Corporation), which contained glycerol polyglycidyl ether, was added. The obtained mixture was stirred at 40°C for 4 hours. After the reaction, while carrying out suction filtration, the beads were washed with distilled water in a 20 times or more volume than that of beads. The obtained beads were referred to as "one-time crosslinked porous cellulose beads".
The obtained one-time crosslinked porous cellulose beads were transferred to a vessel. Distilled water was added thereto such that the total volume became 10 times by volume of the crosslinked porous cellulose beads. The mixture was heated up to 120°C for 1 hour using an autoclave. After the mixture was cooled down to room temperature, the beads were washed with RO water in a 5 times or more volume than that of the beads, to obtain autoclaved one-time crosslinked porous cellulose beads of which an epoxy group was changed to a glyceryl group.
Next, distilled water was added to 11 parts by volume of the autoclaved one-time crosslinked porous cellulose beads such that the total volume became 16.5 parts by volume, and the mixture was transferred to a reaction vessel. 3.86 parts by volume of 4N NaOH aqueous solution which was prepared from NaOH manufactured by NACALAI TESQUE, INC. and distilled water was added thereto. The mixture was heated up to 40°C. A crosslinking agent (DENACOL EX-314, manufactured by Nagase ChemteX Corporation) of 1.77 parts by weight was added thereto. The obtained mixture was stirred at 40°C for 4 hours. After the reaction, the beads were washed with distilled water in 20 times or more volume than that of the beads with carrying out suction filtration. The obtained beads were referred to as "two-times crosslinked porous cellulose beads".
The obtained two-times crosslinked porous cellulose beads were transferred to a vessel. Distilled water was added thereto such that the total volume became 10 times by volume of the crosslinked porous cellulose beads. The mixture was heated up to 120°C for 60 minutes using an autoclave. After the mixture was cooled down to room temperature, the beads were washed using 5 times or more by volume of distilled water, to obtain autoclaved two-times crosslinked porous cellulose beads.

(5) Physical property test of crosslinked porous cellulose beads

The median particle diameter of the above crosslinked porous cellulose beads was 75 $\mu$m. Further, the average pore diameter was 215 Å, maximum pore diameter was 1756 Å, and exclusion limit molecular weight was $5.0 \times 10^8$. Specific surface area was $7.17 \times 10^7$ m$^2$/m$^3$ and a saturated adsorption capacity for IgG in theory was 79 g/L.

Manufacture Example 2

Preparation of non-oriented protein A

Non-oriented protein A used in the present invention had an amino acid sequence shown in SEQ ID: 1. The amino acid sequence was corresponded to an amino acid sequence in which signal sequence (S domain) and cell wall binding domain (X domain) were removed from an amino acid sequence of Staphylococcus aureus protein A, which was described as SPA' in WO 2006/004067. The non-oriented protein A was prepared according to a method described in Example of WO2006/004067. The entire content of WO2006/00406 is incorporated herein for reference.

Example 1

An adsorbent immobilized with non-oriented protein A was prepared according to the following procedures. RO water was added to 11.0 mL of crosslinked porous cellulose beads obtained in Manufacture Example 1 so that a total volume was 17.0 mL. A mixture was transferred to 50 mL of a centrifuge tube, and the tube was set on a mix rotor (manufactured by AS ONE corporation, mix rotor MR-3) at 25 °C, to stir the mixture. Then, 6.0 mL of 8.64 mg/mL sodium periodate solution was prepared by dissolving sodium periodate (manufactured by Wako Pure Chemical Industries, Ltd.) into RO water. The solution was added to the centrifuge tube contained the crosslinked porous cellulose beads to stir for one hour at 25 °C. After reaction, the mixture was washed with RO water on glass filter (11GP 100, manufactured by SIBATA SCIENTIFIC TECHNOLOGY LTD.) such that electric conductivity of a filtrate was 1 $\mu$S/cm or less, to obtain crosslinked porous cellulose beads containing a formyl group. The electric conductivity of the washed filtrate was measured by a conductivity meter (ECTester (registered trademark) 10 Pure+, manufactured by EUTECH INSTRUMENTS).

9.0 mL of the obtained crosslinked porous cellulose beads containing a formyl group was substituted with 30 mL of a buffer containing 0. 5 M trisodium citrate dihydrate (manufactured by KANTO CHEMICAL CO., INC.) and 0.15 M sodium chloride (manufactured by KANTO CHEMICAL CO., INC.) at pH 8 on a glass filter (11GP 100, manufactured by SIBATA SCIENTIFIC TECHNOLOGY LTD.). The crosslinked porous cellulose beads containing a formyl group after substitution was put into a centrifugal tube by using a buffer (0.5 M trisodium citrate dihydrate, 0.15 M sodium chloride) at pH 8, and the total volume was adjusted to be 14.0 mL. After 5.327 g of 67.58 mg/mL non-oriented protein A solution produced in the Manufacture Example 2 was added thereto, pH was adjusted to be 12 by using 0.08 N NaOH (prepared from NaOH produced by Nacalai Tesque, Inc. and RO water) at 6° C. Then, the mixture was stirred at 6° C for 23 hours by using a mix rotor (Mix Rotor MR-3, manufactured by AS ONE Corporation) to progress a reaction.

After the reaction for 23 hours, pH of the reaction mixture was adjusted to be 5.0 by using 2.4 N citric acid (prepared from citric acid produced by KANTO CHEMICAL CO., INC. and RO water), and then the mixture was stirred at 6° C for 4 hours by using a mix rotor (Mix Rotor MR-3, manufactured by AS ONE Corporation). Continuously, 0.39 mL of a 5. 5% dimethylamine borane (DMAB) aqueous solution (prepared from dimethylamine borane produced by Kishida Chemical Co., Ltd. and RO water) was added thereto, and the mixture was stirred at 6 °C for 1 hour. Then, the reaction temperature was increased to 25 °C and the mixture was stirred at 25 °C for 18 hours using a mix rotor (Mix Rotor MR-3, manufactured by AS ONE Corporation) to carry out a reaction. After the completion of the reaction, the maximum UV absorbance of the reaction mixture around 278 nm was measured to obtain an introduced amount of the protein A.

The beads after the reaction was washed with RO water in a 3 times more volume than that of the porous carrier on a glass filter (11GP 100, manufactured by SIBATA SCIENTIFIC TECHNOLOGY LTD.). Next, 0.1 N citric acid solution (prepared from citric acid monohydrate produced by KANTO CHEMICAL CO., INC. and RO water) in a 3 times volume was added, and further 0.1 N citric acid monohydrate was added to the beads to adjust the total volume to be 30 mL or more. The mixture was put into a centrifugal tube and washed with an acid while stirring at 25° C for 30 minutes.

After washing with an acid, the beads were washed with RO water in a 3 times more volume than that of the beads on a glass filter (11GP 100, manufactured by SIBATA SCIENTIFIC TECHNOLOGY LTD.), and thereafter an aqueous solution of 0.05 M sodium hydroxide and 1 M sodium sulfate (prepared from sodium hydroxide produced by Nacalai Tesque, Inc., sodium sulfate produced by KANTO CHEMICAL CO., INC., and RO water) was added in a 3 times more volume than that of the beads. Next, an aqueous solution of 0.05 M sodium hydroxide and 1 M sodium sulfate was added to the beads so that the total volume was adjusted to be 30 mL or more. The mixture was put into a centrifugal tube and washed with an alkali while stirring at room temperature for 30 minutes.

After washing with an alkali, the beads were washed with RO water in a 20 times more volume than that of the beads on a glass filter (11GP 100, manufactured by SIBATA SCIENTIFIC TECHNOLOGY LTD.). Next, 0.5 N trisodium citrate aqueous solution (prepared from trisodium citrate dihydrate produced by KANTO CHEMICAL CO., INC. and RO water) in a 3 times more volume than that of the beads was added, and it was confirmed that the filtrate became neutral. Then, the beads were washed by using RO water until the electric conductivity of the washed filtrate became not more than 1 $\mu$S/cm, to obtain an adsorbent as a target adsorbent on which a protein A was immobilized. The electric conductivity of the washed filtrate was measured by a conductivity meter (ECTester (registered trademark) 10 Pure+, manufactured by EUTECH INSTRUMENTS). Physical properties of beads were evaluated according to Test Examples 8 to 10 as for the adsorbent immobilized with obtained protein A. The results are shown as follows.

Introduced amount of protein A: 35 g/L (a volume of an adsorbent)
5% DBC for RT of 3 minutes: 43 g/L (a volume packed with an adsorbent)
5% DBC for RT of 6 minutes: 49 g/L (a volume packed with an adsorbent)
5% DBC for RT of 9 minutes: 51 g/L (a volume packed with an adsorbent)

Manufacture Example 3

Porous cellulose beads were obtained in a similar condition to the Manufacture Example 1 except that 15 % by weight of citric acid monohydrate-containing methanol solution was used as a coagulating solvent. As shown in Fig. 2, it was confirmed that good pores were formed on the surface of the beads. Next, the beads were subjected to classification as in Manufacture Example 1 to obtain crosslinked porous cellulose beads. The median particle diameter of porous cellulose beads was 75 $\mu$m, an average pore size was 190 Å, maximum pore size was 718 Å, and exclusion limit molecular weight was $3.2 \times 10^7$. In addition, specific surface area was $1.04 \times 10^8$ m$^2$/m$^3$, and a saturated adsorption capacity for IgG in theory was 114 g/L.

Example 2

An adsorbent immobilized with protein A was obtained with crosslinked porous cellulose beads prepared in Manufacture Example 3 in the same manner as in Example 1. Physical properties of the adsorbent were evaluated. The results are shown below. Introduced amount of protein A: 33 g/L (a volume of an adsorbent) 5% DBC for RT of 3 minutes: 43 g/L (a volume packed with an adsorbent)

Manufacture Example 4

(1) Preparation of porous cellulose beads

Porous cellulose beads were prepared in the same manner as in Manufacture Example 1 except that stirring speed was changed to 500 rpm (Pv value: 1.1 kW/m$^3$). As shown in Fig. 3, it was confirmed that good pores were formed on the surface of the beads. The beads were subjected to classification except that 90 $\mu$m of a sieve was changed to 63 $\mu$m of a sieve in the same manner as in Manufacture Example 1.

(2) Crosslinking - Method B

Distilled water was added to 20 parts by volume of the above porous cellulose beads so that the total volume was 30 parts by volume. The mixture was transferred to a reaction vessel. To the mixture, 2.3 parts by weight of a crosslinking agent (DENACOL EX-314, manufactured by Nagase ChemteX Corporation), which contained glycerol polyglycidyl ether, was added. The mixture was stirred with heating up to 40°C. After the temperature became 40°C, the mixture was stirred for 30 minutes. Separately, 7.1 parts by volume of 2N NaOH aqueous solution was prepared from NaOH manufactured by NACALAI TESQUE, INC. and distilled water. The NaOH aqueous solution was added to the above mixture in increments of one quarter per one hour. During the addition, the temperature was maintained at 40°C and stirring was maintained. After final amount of one quarter was added, the mixture was stirred at the same temperature for 1 hour. After the reaction, the beads were washed using 20 times by volume of distilled water while carrying out suction filtration. The obtained porous cellulose beads were referred to as "one-time crosslinked porous cellulose beads".

The obtained one-time crosslinked porous cellulose beads were transferred to a vessel. Distilled water was added thereto so that the total volume became 10 times by volume of the crosslinked porous cellulose beads. The mixture was heated up to 120°C for 1 hour using an autoclave. After the mixture was cooled down to room temperature, the beads were washed with RO water of 5 times or more volume, to obtain autoclaved one-time crosslinked porous cellulose beads of which an epoxy group was changed to a glyceryl group.

Next, distilled water was added to 20 parts by volume of the autoclaved one-time crosslinked porous cellulose beads so that the total volume became 30 parts by volume. The mixture was transferred to a reaction vessel, and 2.3 parts by weight of a crosslinking agent (DENACOL EX-314, manufactured by Nagase ChemteX Corporation), which contained glycerol polyglycidyl ether, was added thereto. The mixture was heated up to 40°C with stirring. After the temperature became 40°C, the mixture was stirred for 30 minutes. Separately, 7.1 parts by volume of 2N NaOH aqueous solution was prepared from NaOH manufactured by NACALAI TESQUE, INC. and distilled water. The NaOH aqueous solution was added to the above mixture in increments of one quarter per one hour. During the addition, the temperature was maintained at 40°C and stirring was maintained. After final amount of one quarter was added, the mixture was stirred at the same temperature for 1 hour. After the reaction, the beads were washed with distilled water in a 20 times or more volume than that of the beads with carrying out suction filtration. The obtained beads were referred to as "two-times crosslinked porous cellulose beads".

The obtained two-times crosslinked porous cellulose beads were transferred to a vessel. Distilled water was added thereto so that the total volume became 10 times by volume of the crosslinked porous cellulose beads. The mixture was heated up to 120°C for 60 minutes using an autoclave. After the mixture cooled down to room temperature, the beads were washed with distilled water of 5 times or more volume, to obtain autoclaved two-times crosslinked porous cellulose beads.

(3) Physical properties test of crosslinked porous cellulose beads

The median particle diameter of the porous cellulose beads was 56 $\mu$m, an average pore size was 336 Å, maximum pore size was 3400 Å, and exclusion limit molecular weight was $3.8 \times 10^9$. In addition, specific surface area was $6.88 \times 10^7$ m$^2$/m$^3$, and a saturated adsorption capacity for IgG in theory was 75 g/L. The beads were not critically compressed even at a linear velocity of 3057 cm/h.

Example 3

An adsorbent immobilized with protein A was obtained with crosslinked porous cellulose beads prepared in Manufacture Example 4 in the same manner as in Example 1. Physical properties of the adsorbent were evaluated. The results are shown below. Introduced amount of protein A: 36 g/L (a volume of an adsorbent) 5% DBC for RT of 3 minutes: 70 g/L (a volume packed with an adsorbent)

Manufacture Example 5

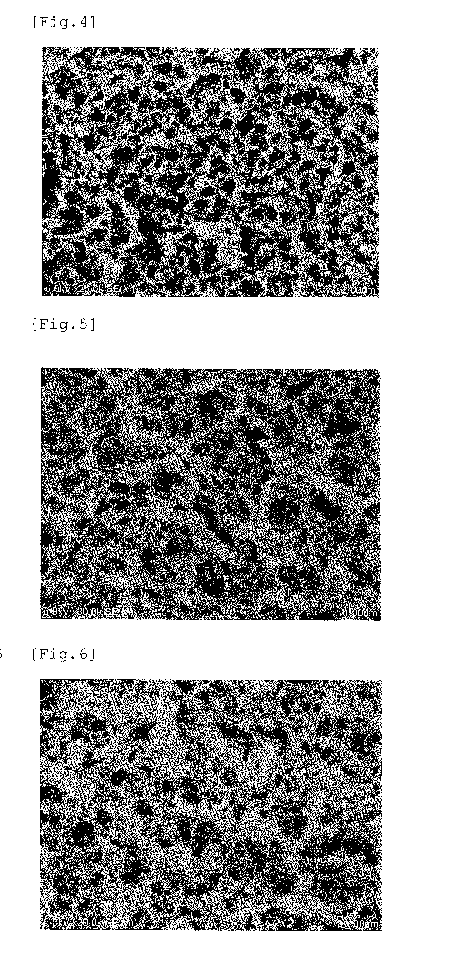

Porous cellulose beads were prepared in the same manner as in Manufacture Example 1 except that the coagulate solvent was changed to 15 % by weight of citric acid monohydrate-containing ethanol solution. As shown in Fig. 4, it was confirmed that good pores were formed on the surface of the beads. The beads were subjected to classification in the same manner as in Manufacture Example 1 to obtain crosslinked porous cellulose beads. The median particle diameter of the porous cellulose beads was 75 $\mu$m, an average pore size was 163 Å, maximum pore size was 1040 Å, and exclusion limit molecular weight was $1.0 \times 10^8$. In addition, specific surface area was $7.85 \times 10^7$ m$^2$/m$^3$, and a saturated adsorption capacity for IgG in theory was 86 g/L.

Example 4

An adsorbent immobilized with protein A was obtained with crosslinked porous cellulose beads prepared in Manufacture Example 5 in the same manner as in Example 1. Physical properties of the adsorbent were evaluated. The results are shown below. Introduced amount of protein A: 36 g/L (a volume of an adsorbent) 5% DBC for RT of 3 minutes: 8 g/L (a volume packed with an adsorbent)

Manufacture Example 6

Porous cellulose beads were obtained in a similar condition to the Manufacture Example 4 except that a sieve of 90 $\mu$m was used instead of a sieve of 63 $\mu$m. As shown in Fig. 5, it was confirmed that good pores were formed on the surface of the beads. The beads were subjected to classification in the same manner as in Manufacture Example 4, to obtain crosslinked porous cellulose beads. The median particle diameter of the obtained crosslinked cellulose beads was 75 $\mu$m. The beads were not critically compressed even at a linear velocity of 3057 cm/h, which was the maximum linear velocity of a passing liquid in the used device.

Example 5

An adsorbent immobilized with protein A was obtained with crosslinked porous cellulose beads prepared in Manufacture Example 6 in the same manner as in Example 1. Physical properties of the adsorbent were evaluated. The results are shown below. Introduced amount of protein A: 36 g/L (a volume of an adsorbent) 5% DBC for RT of 3 minutes: 42 g/L (a volume packed with an adsorbent)

Manufacture Example 7

Porous cellulose beads were obtained in a similar condition to the Manufacture Example 6 except that a rotational ratio for stirring was 700 rpm (Pv value: 3.1 kW/m$^3$). As shown in Fig. 6, it was confirmed that good pores were formed on the surface of the beads. The beads were subjected to classification in the same manner as in Manufacture Example 6, to obtain crosslinked porous cellulose beads. Median particle diameter of the obtained crosslinked porous cellulose beads was 75 μm. The beads were not critically compressed even at a linear velocity of 3057 cm/h.

Example 6

An adsorbent immobilized with protein A was obtained with crosslinked porous cellulose beads prepared in Manufacture Example 7 in the same manner as in Example 1. Physical properties of the adsorbent were evaluated. The results are shown below. Introduced amount of protein A: 38 g/L (a volume of an adsorbent) 5% DBC for RT of 3 minutes: 49 g/L (a volume packed with an adsorbent)

Manufacture Example 8

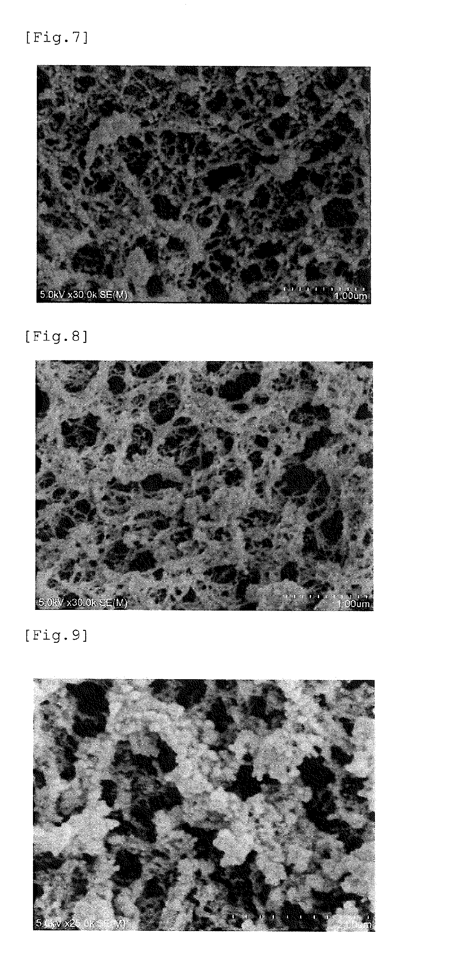

Porous cellulose beads were obtained in a similar condition to the Manufacture Example 6 except that a rotational ratio for stirring was 250 rpm (Pv value: 0.1 kW/m$^3$). As shown in Fig. 7, it was confirmed that good pores were formed on the surface of the beads. The beads were subjected to classification in the same manner as in Manufacture Example 6, to obtain crosslinked porous cellulose beads. The median particle diameter of the obtained crosslinked porous cellulose beads was 75 μm, the average pore diameter was 130 Å, maximum pore diameter was 562 Å, and exclusion limit molecular weight was $1.5 \times 10^7$. In addition, specific surface area was $8.72 \times 10^7$ m$^2$/m$^3$, and a saturated adsorption capacity for IgG in theory was 96 g/L.

Example 7

An adsorbent immobilized with protein A was obtained with crosslinked porous cellulose beads prepared in Manufacture Example 8 in the same manner as in Example 1. Physical properties of the adsorbent were evaluated. The results are shown below. Introduced amount of protein A: 37 g/L (a volume of an adsorbent) 5% DBC for RT of 3 minutes: 36 g/L (a volume packed with an adsorbent)

Manufacture Example 9

Porous cellulose beads were obtained in a similar condition to the Manufacture Example 6 except that one large-size blade having two H like parts (in the present specification, referred to as WH type large-size blade) shown in Fig. 16 was used as a stirring blade and a rotational ratio for stirring was 350 rpm (Pv value: 1.1 kW/m$^3$). As shown in Fig. 8, it was confirmed that good pores were formed on the surface of the beads. The beads were subjected to classification in the same manner as in Manufacture Example 6, to obtain crosslinked porous cellulose beads. Particle size distribution after granulation was wide compared with that of Example 5. The median particle diameter of the obtained crosslinked porous cellulose beads was 75 μm, the average pore diameter was 418 Å, maximum pore diameter was 1137 Å, and exclusion limit molecular weight was $1.3 \times 10^8$. In addition, specific surface area was $8.38 \times 10^7$ m$^2$/m$^3$, and a saturated adsorption capacity for IgG in theory was 92 g/L.

Example 8

An adsorbent immobilized with protein A was obtained with crosslinked porous cellulose beads prepared in Manufacture Example 9 in the same manner as in Example 1. Physical properties of the adsorbent were evaluated. The results are shown below. Introduced amount of protein A: 35 g/L (a volume of an adsorbent) 5% DBC for RT of 3 minutes : 29 g/L (a volume packed with an adsorbent)

Manufacture Example 10

Porous cellulose beads were obtained in a similar condition to the Manufacture Example 6 except that time needed to add a coagulate solvent was 60 seconds. As shown in Fig. 9, it was confirmed that good pores were formed on the surface of the beads. The beads were subjected to classification in the same manner as in Manufacture Example 6, to obtain crosslinked porous cellulose beads. The median particle diameter of the obtained crosslinked porous cellulose beads was 75 $\mu$m, the average pore diameter was 194 Å, maximum pore diameter was 747 Å, and exclusion limit molecular weight was $3.7 \times 10^7$. In addition, specific surface area was $1.02 \times 10^8$ m$^2$/m$^3$, and a saturated adsorption capacity for IgG in theory was 112 g/L.

Example 9

An adsorbent immobilized with protein A was obtained with crosslinked porous cellulose beads prepared in Manufacture Example 10 in the same manner as in Example 1. Physical properties of the adsorbent were evaluated. The results are shown below. Introduced amount of protein A: 31 g/L (a volume of an adsorbent) 5% DBC for RT of 3 minutes: 55 g/L (a volume packed with an adsorbent)

Manufacture Example 11

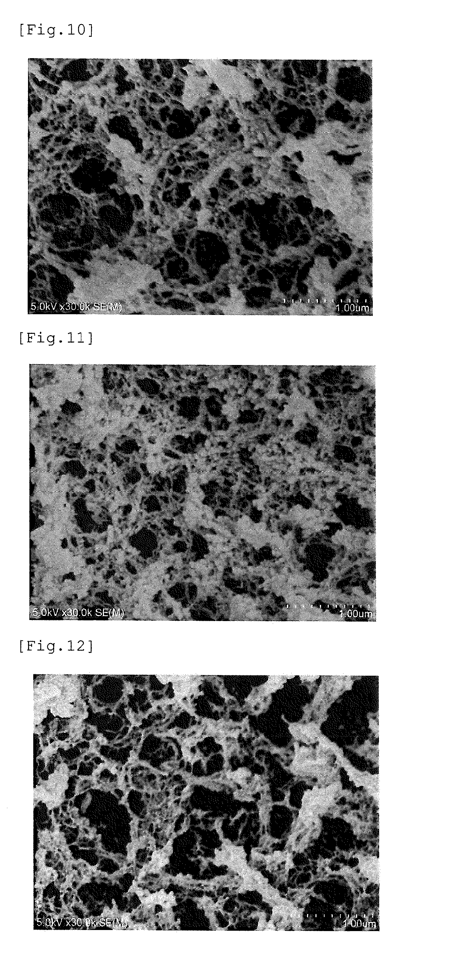

Porous cellulose beads were obtained in a similar condition to the Manufacture Example 7 except that a stirring blade was two pieces of pitched paddle blades. As shown in Fig. 10, it was confirmed that good pores were formed on the surface of the beads. The beads were subjected to classification in the same manner as in Manufacture Example 7, to obtain crosslinked porous cellulose beads. The median particle diameter of the obtained crosslinked porous cellulose beads was 75 $\mu$m, the average pore diameter was 221 Å, maximum pore diameter was 2407 Å, and exclusion limit molecular weight was $1.3 \times 10^9$. In addition, specific surface area was $6.42 \times 10^7$ m$^2$/m$^3$, and a saturated adsorption capacity for IgG in theory was 70 g/L.

Example 10

An adsorbent immobilized with protein A was obtained with crosslinked porous cellulose beads prepared in Manufacture Example 11 in the same manner as in Example 1. Physical properties of the adsorbent were evaluated. The results are shown below. Introduced amount of protein A: 34 g/L (a volume of an adsorbent) 5% DBC for RT of 3 minutes: 36 g/L (a volume packed with an adsorbent)

Manufacture Example 12

Porous cellulose beads were obtained in a similar condition to the Manufacture Example 11 except that a temperature to be adjusted was 9 °C. As shown in Fig. 11, it was confirmed that good pores were formed on the surface of the beads. The beads were subjected to classification in the same manner as in Manufacture Example 11, to obtain crosslinked porous cellulose beads.

Example 11

An adsorbent immobilized with protein A was obtained with crosslinked porous cellulose beads prepared in Manufacture Example 12 in the same manner as in Example 1. Physical properties of the adsorbent were evaluated. The results are shown below. Introduced amount of protein A: 34 g/L (a volume of an adsorbent) 5% DBC for RT of 3 minutes: 22 g/L (a volume packed with an adsorbent)

Manufacture Example 13

Porous cellulose beads were obtained in a similar condition to the Manufacture Example 11 except that a temperature was adjusted to 0 °C. As shown in Fig. 12, it was confirmed that good pores were formed on the surface of the beads. The beads were subjected to classification in the same manner as in Manufacture Example 11, to obtain crosslinked porous cellulose beads.

Example 12

An adsorbent immobilized with protein A was obtained with crosslinked porous cellulose beads prepared in Manufacture Example 13 in the same manner as in Example 1. Physical properties of the adsorbent were evaluated. The results are shown below. Introduced amount of protein A: 35 g/L (a volume of an adsorbent) 5% DBC for RT of 3 minutes: 14 g/L (a volume packed with an adsorbent)

Manufacture Example 14

(1)Preparation of cellulose dispersion B

76 g of Japanese Pharmacopoeia cellulose PH-F20JP, which was manufactured by Asahi Kasei Chemicals Corporation and of which median particle diameter was 21 $\mu$m, and 800 g of distilled water were mixed. The temperature of the mixture was adjusted to 4°C with stirring. To the stirred mixture, 400 g of the alkaline aqueous solution A of which temperature was adjusted to 4°C was added while maintaining a preset temperature and stirring. The obtained mixture was stirred for 30 minutes.

(2) Preparation of porous cellulose beads

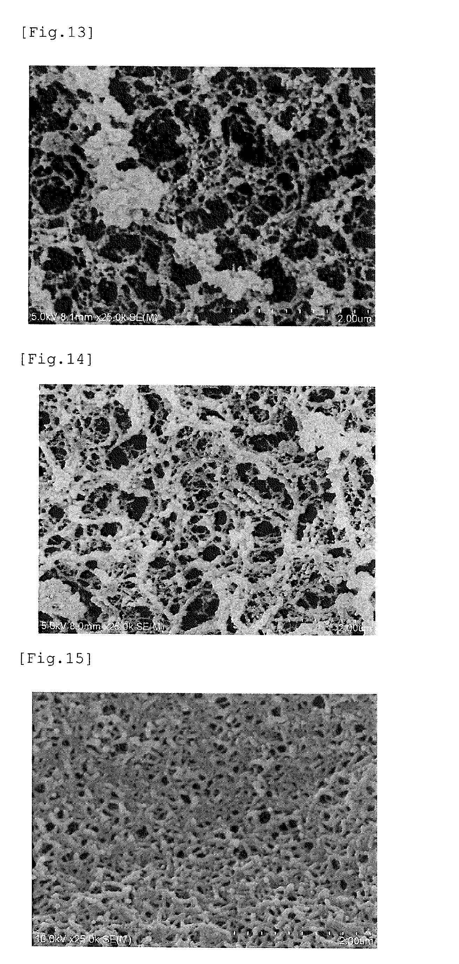

The cellulose dispersion B, orthodichlorobenzene and sorbitan monooleate (corresponding to span 80) of which temperatures were adjusted to 4°C were mixed in a ratio of 1276 g, 7801 g and 78 g respectively. The mixture was stirred in a stainless vessel equipped with two rushton turbine blades at 460 rpm (Pv value: 5.0 kW/m$^3$) at 4°C for 15 minutes, to prepare an emulsion. To the emulsion, 592 g of methanol of which temperature was adjusted to 4°C was added as a coagulating solvent with stirring and maintaining the temperature. The time required for adding the coagulating solvent was 5 seconds. Then, the mixture was stirred for 30 minutes while maintaining the rotational rate for stirring and the temperature. After pressure filtration was carried out, the mixture was washed using 3000 g of ethanol and subsequently 3000 g of water to obtain porous cellulose beads. As shown in Fig. 13, it was confirmed that good pores were formed on the surface of the beads. The obtained porous cellulose beads were subjected to wet-classification using sieves of 38 $\mu$m and 90 $\mu$m in the same manner as in Manufacture Example 1.
Then, crosslinked porous cellulose beads were obtained in the same manner as in Manufacture Example 4. The median particle diameter was 75 $\mu$m.

Example 13

An adsorbent immobilized with protein A was obtained with crosslinked porous cellulose beads prepared in Manufacture Example 14 in the same manner as in Example 1. Physical properties of the adsorbent were evaluated. The results are shown below. Introduced amount of protein A: 35 g/L (a volume of an adsorbent) 5% DBC for RT of 3 minutes: 57 g/L (a volume packed with an adsorbent)

Manufacture Example 15

Porous cellulose beads were obtained in the same manner as in Manufacture Example 14 except that 1212 g of the cellulose dispersion B, 8238 g of orthodichlorobenzene, 85 g of sorbitan monooleate (corresponding to span 80), and 740 g of methanol as a coagulate solvent were used. As shown in Fig. 14, it was confirmed that good pores were formed on the surface of the beads. The obtained porous cellulose beads were subjected to classification and crosslinking in the same manner as in Manufacture Example 14.

Example 14

An adsorbent immobilized with protein A was obtained with crosslinked porous cellulose beads prepared in Manufacture Example 15 in the same manner as in Example 1. Physical properties of the adsorbent were evaluated. The results are shown below. Introduced amount of protein A: 30 g/L (a volume of an adsorbent) 5% DBC for RT of 3 minutes: 60 g/L (a volume packed with an adsorbent)

Manufacture Example 16

Preparation of orientation-controlled protein A

An orientation-controlled protein A used in the present invention had an amino acid sequence shown in SEQ ID: 2. The orientation-controlled protein A had a structure that four C domain variants in which 4th Lys, 7th Lys, 35th Lys, 42nd Lys, 49th Lys, 50th Lys, and 58th Lys of C domain were substituted with Arg, and 29th Gly was substituted with Ala were connected (Lys at C-terminal was not substituted). The orientation-controlled protein A was prepared according to C domain variant and a method for preparing a connected body thereof described in WO2011/118699. The entire content of WO2011/118699 was incorporated herein for reference.

Example 15

3.5 mL of crosslinked porous cellulose beads obtained in Manufacture Example 14 was charged into a centrifuge tube. To the centrifuge tube, RO water was added so that a total volume was 6 mL. The tube was set on a mix rotor (manufactured by AS ONE corporation, mix rotor MR-3) at 25 °C, to stir the mixture. Then, 2.0 mL of 11.16 mg/mL sodium periodate aqueous solution prepared by dissolving sodium periodate (Wako Pure Chemical Industries, Ltd.) into RO water was added to stir for one hour at 25 °C. After reaction, the mixture was washed with RO water on glass filter (11GP 100, manufactured by SIBATA SCIENTIFIC TECHNOLOGY LTD.) so that electric conductivity of a filtrate was 1 $\mu$S/cm or less, to obtain crosslinked porous cellulose beads containing a formyl group. The electric conductivity of the washed filtrate was measured by a conductivity meter (ECTester (registered trademark) 10 Pure+, manufactured by EUTECH INSTRUMENTS).

3.5 mL of the obtained crosslinked porous cellulose beads containing a formyl group was substituted with 0.6 M of citrate buffer (prepared with trisodium citrate dihydrate produced by Wako Pure Chemical Industries, Ltd., sodium hydroxide and RO water) at pH 12 on a glass filter (11GP 100, manufactured by SIBATA SCIENTIFIC TECHNOLOGY LTD.). The crosslinked porous cellulose beads containing a formyl group after substitution were put into a centrifugal tube by using 0.6 M of citrate buffer at pH 12, and the total volume was adjusted to be 7.5 mL. To the tube, 0.82 g of an aqueous solution (protein A concentration of 63.7 mg/mL) containing orientation-controlled protein A prepared in Manufacture Example 16 was added, and the mixture was subjected to a reaction at 6 °C for 23 hours with stirring by using a mix rotor (Mix Rotor MR-3, manufactured by AS ONE Corporation).

After the reaction, a reaction liquid was collected (reaction liquid 1), and substituted with 0.1 M sodium citrate solution (prepared from trisodium citrate dihydrate produced by Wako Pure Chemical Industries, Ltd. and RO water) at pH 8, and then the mixture was stirred at 6° C for 4 hours by using a mix rotor (Mix Rotor MR-3, manufactured by AS ONE Corporation). Continuously, 1.93 mL of 5.5% by weight of dimethylamine borane (DMAB) aqueous solution (prepared from dimethylamine borane produced by Wako Pure Chemical Industries, Ltd. and RO water) was added thereto, and the mixture was stirred at 6 °C for 1 hour. Then, the reaction temperature was increased to 25 °C and the mixture was stirred at 25 °C for 18 hours using a mix rotor (Mix Rotor MR-3, manufactured by AS ONE Corporation) to make the mixture reacted. After the completion of the reaction, a reaction liquid was collected (reaction liquid 2). The maximum UV absorbances around 278 nm of the reaction liquids 1 and 2 were measured to obtain an immobilized amount of the protein A by subtracting obtained absorbance from absorbance corresponding to a charged amount of a ligand.

The beads after the reaction were washed with RO water in a 3 times more volume than that of the porous beads on a glass filter (11GP 100, manufactured by SIBATA SCIENTIFIC TECHNOLOGY LTD.). Next, 0.1 N citric acid monohydrate solution (prepared from citric acid monohydrate produced by KANTO CHEMICAL CO. , INC. and RO water) in a 3 times more volume was added, and further 0.1 N citric acid monohydrate was added to the beads to adjust the total volume to be 30 mL or more. The mixture was put into a centrifugal tube and washed with an acid while stirring at 25° C for 30 minutes.

After washing with an acid, the beads were washed with RO water in a volume of 3 times as much as that of the beads on a glass filter (11GP 100, manufactured by SIBATA SCIENTIFIC TECHNOLOGY LTD.), and thereafter an aqueous solution of 0.05 M sodium hydroxide and 1 M sodium sulfate (prepared from sodium hydroxide produced by Nacalai Tesque, Inc., sodium sulfate produced by KANTO CHEMICAL CO., INC. , and RO water) was added in a 3 times more volume relative to the volume of the beads. Next, an aqueous solution of 0.05 M sodium hydroxide and 1 M sodium sulfate was added to the beads so that the total volume was adjusted to be 30 mL or more. The mixture was put into a centrifugal tube and washed with an alkali while stirring at room temperature for 30 minutes.

After washing with an alkali, the beads were washed with RO water in a 20 times more volume than that of the beads on a glass filter (11GP 100, manufactured by SIBATA SCIENTIFIC TECHNOLOGY LTD.). Next, 0.1 N sodium citrate solution (prepared from trisodium citrate dihydrate produced by KANTO CHEMICAL CO., INC. and RO water) in a 3 times more volume than that of the beads was added, and it was confirmed that the filtrate became neutral. Then, the beads were washed by using RO water until the electric conductivity of the washed filtrate became not more than 1 $\mu$S/cm, to obtain an adsorbent on which protein A to be introduced was immobilized. The electric conductivity of the washed filtrate was measured by a conductivity meter (ECTester (registered trademark) 10 Pure+, manufactured by EUTECH INSTRUMENTS).

Physical evaluation of beads was carried out according to Test Examples 8 to 9 as for the adsorbent immobilized with obtained protein A. The results are shown as follows.

Introduced amount of protein A: 9 g/L (a volume of an adsorbent)

5% DBC for RT of 3 minutes: 56 g/L (a volume packed with an adsorbent)

5% DBC for RT of 6 minutes: 64 g/L (a volume packed with an adsorbent)

Example 16

An adsorbent immobilized with protein A was obtained in the same manner as in Example 15 except that an added amount of a ligand was 1.64 mL. Physical properties of the adsorbent were evaluated. The results are shown below. Introduced amount of protein A: 17 g/L (a volume of an adsorbent) 5% DBC for RT of 3 minutes: 61 g/L (a volume packed with an adsorbent) 5% DBC for RT of 6 minutes: 79 g/L (a volume packed with an adsorbent)

Comparative Manufacture Example 1

(1) Preparation of cellulose solution

To 100 g of 60 wt% calcium thiocyanate aqueous solution, 6.4 g of crystalline cellulose (CEOLUS PH101, manufactured by Asahi Kasei Chemicals Corporation, median particle diameter: 73 $\mu$m) was added. The mixture was heated up to 120°C to dissolve cellulose. The solution was used shortly after the preparation, since it is difficult to preserve the solution at 120°C.

(2) Preparation of crosslinked porous cellulose beads

Porous cellulose beads were prepared using calcium thiocyanate with reference to the Examples described in WO2010/095673 as follows. Specifically, 6 g of sorbitan monooleate was added as surfactant to the above cellulose solution, and the mixture was added dropwise to 480 mL of orthodichlorobenzene which was preliminarily heated up to 140°C. The mixture was stirred at 300 rpm. Next, the above dispersion was cooled down to 40°C, and poured into 190 mL of methanol to coagulate cellulose. After suction filtration was carried out, a filtrate was washed using 190 mL of methanol. The wash with methanol was repeated several times. After washing was further carried out using a large amount of distilled water, suction filtration was carried out to obtain porous cellulose beads. Porous cellulose beads were filtered, and 100 g of the beads were added to a solution prepared by dissolving 60 g of sodium sulfate in 121 g of distilled water. The mixture was stirred at 50°C for 2 hours. Then, 3.3 g of 45 wt% sodium hydroxide aqueous solution and 0.5 g of sodium borohydride were added thereto, and the mixture was stirred. To the stirred mixture at 50°C, 48 g of 45 wt% sodium hydroxide aqueous solution and 50 g of epichlorohydrin were added in increments of 25 equal parts respectively every 15 minutes. After the addition, the reaction was carried out at 50°C for 16 hours. After the reaction, the mixture was cooled down to 40°C, and neutralized by adding 2.6 g of acetic acid. Suction filtration was carried out and a filtrate was washed using distilled water. Wet classification was carried out using sieves of 53 $\mu$m and 90 $\mu$m to obtain crosslinked porous cellulose beads having average particle diameter of 78 $\mu$m.

(3) Physical property test

The surface pore diameter of the above crosslinked porous cellulose beads was 1649 Å, the average pore diameter was 793 Å, maximum pore diameter was 14100 Å, and exclusion limit molecular weight was $2.9 \times 10^{11}$. The beads were not critically compressed even at a linear velocity of 3057 cm/h. As the above, the crosslinked porous cellulose beads obtained in Comparative Manufacture Example 1 had considerably too large pores. In addition, the solution containing calcium thiocyanate, which was highly toxic, remained as a waste liquid.

Comparative Example 1

An adsorbent immobilized with protein A was obtained with crosslinked porous cellulose beads prepared in Comparative Manufacture Example 1 in the same manner as in Example 1. Physical properties of the adsorbent were evaluated. The results are shown below.
Introduced amount of protein A: 32 g/L (a volume of an adsorbent) 5% DBC for RT of 3 minutes: 41 g/L (a volume packed with an adsorbent)

Comparative Example 2

An adsorbent immobilized with orientation-controlled protein A was obtained in the same manner as in Example 15 except that crosslinked porous cellulose beads prepared in Comparative Manufacture Example 1 was used and a volume of a solution containing orientation-controlled protein A was 0.51 mL. Physical properties of the adsorbent were evaluated. The results are shown below. Introduced amount of protein A: 8 g/L (a volume of an adsorbent) 5% DBC for RT of 3 minutes: 35 g/L (a volume packed with an adsorbent) 5% DBC for RT of 6 minutes: 41 g/L (a volume packed with an adsorbent)

Comparative Example 3

An adsorbent immobilized with orientation-controlled protein A was obtained in the same manner as in Comparative Example 2 except that a volume of a solution containing orientation-controlled protein A was 0.76 mL. Physical properties of the adsorbent were evaluated. The results are shown below.
Introduced amount of protein A: 10 g/L (a volume of an adsorbent) 5% DBC for RT of 3 minutes: 41 g/L (a volume packed with an adsorbent) 5% DBC for RT of 6 minutes: 48 g/L (a volume packed with an adsorbent)

Comparative Example 4

An adsorbent immobilized with orientation-controlled protein A was obtained in the same manner as in Comparative Example 2 except that a volume of a solution containing orientation-controlled protein A was 1.01 mL. Physical properties of the adsorbent were evaluated. The results are shown below.
Introduced amount of protein A: 15 g/L (a volume of an adsorbent) 5% DBC for RT of 3 minutes: 44 g/L (a volume packed with an adsorbent) 5% DBC for RT of 6 minutes: 52 g/L (a volume packed with an adsorbent)

Reference Example 1

The average porous diameter of commercially available crosslinked porous agarose beads (MabSelect SuRe LX, manufactured by GE Healthcare Corp.), of which amount of monoclonal antibody to be adsorbed was relatively large and in which Protein A was introduced, was 425 Å. The maximum pore diameter thereof was 2970 Å, and the exclusion limit molecular weight thereof was $2.5 \times 10^9$.
SEM photograph of the surface of beads was shown in Fig. 15. Performance of adsorption of the beads is as follows. 5% DBC for RT of 3 minutes: 46 g/L (a volume packed with an adsorbent) 5% DBC for RT of 6 minutes: 61 g/L (a volume packed with an adsorbent)

Example 17

(1) Epoxidation

Porous cellulose beads prepared in Example 14 were subjected to wet classification by using 38 μm of a sieve and 150 μm of a sieve. 1 part by volume of RO water was added to 1 part by volume of beads classified, and 0.53 parts by volume of 2N sodium hydroxide solution was added thereto to heat at 45 °C for 30 minutes. Next, 0.18 parts by volume of epichlorohydrin was added to stir a mixture at 45 °C for 2 hours. The mixture was subjected to filtration on glass filter, beads were washed with a large amount of RO water to obtain porous cellulose beads containing an epoxy group. The content of the epoxy group was 17 μmol per 1g of wet weight.

(2) Immobilization of dextran sulfate

26 wt/vol% of dextran sulfate solution (about 18 % sulfate content, molecular weight of about 4000) was added to 0.7 part by volume of porous cellulose beads containing an epoxy group so that a total volume was 1.0 part by volume. Next, 2N sodium hydroxide solution was added thereto to adjust pH to 9.5. A mixture was stirred at 40 °C for 16 hours. The mixture was subjected to filtration on glass filter, and beads were washed with a large amount of RO water, to obtain beads immobilized with dextran sulfate. An introduced amount of dextran sulfate was 14 mg per 1 mL of beads.

(3) Blocking of remaining epoxy group

A remaining epoxy group was blocked by adding 1 part by volume of RO water, 0.25 parts by volume of monoethanolamine to 1 part by volume of beads immobilized with dextran sulfate. A mixture was subjected to filtration on glass filter, and beads were washed with a large amount of RO water to obtain a target adsorbent.

(4) Adsorption test of LDL cholesterol

Human blood was centrifuged at 3000 rpm for 15 minutes to obtain plasma having 93 mg/dL of LDL cholesterol concentration. 3 mL of plasma was added to 0.5 ml of adsorbents washed with physiological saline to shake at 37 °C for 2 hours. An amount of LDL cholesterol of a supernatant after shaking was measured with LDL cholesterol kit (manufacture by SEKISUI MEDICAL CO., LTD. Cholestest (registered trademark) LDL) to obtain an amount of LDL cholesterol adsorbed in adsorbents. Here, in the case where a physiological saline was used in place of beads, LDL concentration of a supernatant was 81 mg/dL, and this value was used in calculation of LDL concentration. It was found that 89% of LDL cholesterol was adsorbed for adsorbents. An adsorption capacity of LDL cholesterol to adsorbents was 5.0 g per 1 L of adsorbents.

Example 18

LDL cholesterol adsorption test was carried out in the same manner as in Example 17 except that an amount of plasma added in Example 17 was 6 mL. Here, in the case where a physiological saline was used in place of beads, LDL concentration of a supernatant was 87 mg/dL, and this value was used in calculation of LDL concentration. As a result, it was found that 63% of LDL cholesterol was adsorbed for adsorbents. An adsorption capacity of LDL cholesterol to adsorbents was 7.0 g per 1 L of adsorbents.

Reference Example 2

LDL cholesterol adsorption test was carried out in the same manner as in Example 17 except that adsorbents packed in adsorption column for plasma purification LIPOSORBER LA-15 (manufactured by KANEKA CORPORATION) were used. As a result, it was found that 80% of LDL cholesterol was adsorbed for adsorbents. An adsorption capacity of LDL cholesterol to adsorbents was 3.9 g per 1 L of adsorbents.

Reference Example 3

LDL cholesterol adsorption test was carried out in the same manner as in Example 18 except that adsorbents packed in adsorption column for plasma purification LIPOSORBER LA-15 (manufactured by KANEKA CORPORATION) were used. As a result, it was found that 53% of LDL cholesterol was adsorbed for adsorbents. An adsorption capacity of LDL cholesterol to adsorbents was 5.5 g per 1 L of adsorbents.

INDUSTRIAL APPLICABILITY

The adsorbent of the present invention can be used in purification and treatment.

**Claims**

1. An adsorbent comprising porous cellulose beads obtained by mixing a cold alkaline aqueous solution and cellulose powder as a raw material to prepare a cellulose dispersion, and bringing the cellulose dispersion into contact with a coagulating solvent.

2. The adsorbent according to claim 1, which comprises an affinity ligand.

3. The adsorbent according to claim 1 or 2, wherein the introduced amount of the affinity ligand is not less than 1 mg and not more than 500 mg per 1 mL of the adsorbent.

4. The adsorbent according to claim 2 or 3, wherein the affinity ligand is protein A.

5. The adsorbent according to claim 4, wherein the protein A has an alkaline resistance.

6. The adsorbent according to claim 5, wherein the protein A is an orientation-controlled protein A.

7. The adsorbent according to any one of claims 1 to 6, wherein 5 % DBC of IgG for residence time of 3 minutes is not less than 60 g/L.

8.  The adsorbent according to any one of claims 1 to 7, wherein 5 % DBC of IgG for residence time of 6 minutes is not less than 70 g/L.

9.  The adsorbent according to any one of claims 1 to 3, which comprises dextran sulfate as a ligand.

10. The adsorbent according to any one of claims 1 to 3 and 9, wherein the adsorption capacity of LDL cholesterol is not less than 7 g/L.

11. The adsorbent according to any one of claims 1 to 10, wherein the temperature of the cold alkaline aqueous solution is not more than 20 °C.

12. The adsorbent according to any one of claims 1 to 11, wherein a cellulose concentration in the cellulose dispersion is not less than 1 wt% and not more than 10 wt%.

13. The adsorbent according to any one of claims 1 to 12, wherein an alkaline concentration in the alkaline aqueous solution is not less than 5 wt% and not more than 15 wt%.

14. A method for purification using the adsorbent according to any one of claims 1 to 13.

15. A method for treatment using the adsorbent according to any one of claims 1 to 13.

[Fig.1]

[Fig.2]

[Fig.3]

[Fig.4]

[Fig.5]

[Fig.6]

[Fig.7]

[Fig.8]

[Fig.9]

[Fig.10]

[Fig.11]

[Fig.12]

[Fig.13]

[Fig.14]

[Fig.15]

[Fig.16]

[Fig.17]

**INTERNATIONAL SEARCH REPORT**

| International application No. |
| --- |
| PCT/JP2013/074184 |

| A. CLASSIFICATION OF SUBJECT MATTER |
| --- |
| *C08B15/10*(2006.01)i, *A61K35/14*(2006.01)i, *A61P3/06*(2006.01)i, *A61P7/04* (2006.01)i, *A61P7/08*(2006.01)i, *A61P9/10*(2006.01)i, *A61P29/00*(2006.01)i, *C08B15/08*(2006.01)i |
| According to International Patent Classification (IPC) or to both national classification and IPC |

| B. FIELDS SEARCHED |
| --- |
| Minimum documentation searched (classification system followed by classification symbols) |
| C08B15/10, A61K35/14, A61P3/06, A61P7/04, A61P7/08, A61P9/10, A61P29/00, C08B15/08 |

| Documentation searched other than minimum documentation to the extent that such documents are included in the fields searched |
| --- |
| Jitsuyo Shinan Koho 1922–1996 Jitsuyo Shinan Toroku Koho 1996–2013 Kokai Jitsuyo Shinan Koho 1971–2013 Toroku Jitsuyo Shinan Koho 1994–2013 |

| Electronic data base consulted during the international search (name of data base and, where practicable, search terms used) |
| --- |
| JSTPlus/JMEDPlus/JST7580(JDreamIII) |

| C. DOCUMENTS CONSIDERED TO BE RELEVANT | | |
| --- | --- | --- |
| Category* | Citation of document, with indication, where appropriate, of the relevant passages | Relevant to claim No. |
| X<br>Y | JP 2011-231152 A (JNC Corp.),<br>17 November 2011 (17.11.2011),<br>claims 1 to 8; examples 1 to 7<br>(Family: none) | 1-8,11-14<br>4-10 |
| X<br>Y | JP 2009-14377 A (Tosoh Corp.),<br>22 January 2009 (22.01.2009),<br>claim 1; paragraphs [0002], [0045]; example 2<br>(Family: none) | 1-3,11-14<br>4-10 |
| X<br>Y | JP 11-158202 A (Kaneka Corp.),<br>15 June 1999 (15.06.1999),<br>claims 1 to 9; paragraphs [0009], [0050],<br>[0074]; examples 1 to 5<br>& US 2003/0012941 A1 & US 2003/0186041 A1<br>& US 2008/0070027 A1 & EP 955332 A1<br>& EP 1693402 A2 & WO 1998/030620 A1 | 1-3,11-14<br>4-10 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 31 October, 2013 (31.10.13) | 12 November, 2013 (12.11.13) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**INTERNATIONAL SEARCH REPORT**

| International application No. |
| --- |
| PCT/JP2013/074184 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | JP 10-248927 A  (Kaneka Corp.),<br>22 September 1998 (22.09.1998),<br>example 1<br>& US 2003/0012941 A1     & US 2003/0186041 A1<br>& US 2008/0070027 A1     & EP 955332 A1<br>& EP 1693402 A2           & WO 1998/030620 A1 | 9-10 |
| Y | JP 4-227268 A  (Kaneka Corp.),<br>17 August 1992 (17.08.1992),<br>example 2<br>(Family: none) | 9-10 |
| P,X | WO 2012/121258 A1  (Kaneka Corp.),<br>13 September 2012 (13.09.2012),<br>entire text<br>(Family: none) | 1-8,11-14 |

Form PCT/ISA/210 (continuation of second sheet) (July 2009)

**INTERNATIONAL SEARCH REPORT**

| International application No. |
| --- |
| PCT/JP2013/074184 |

**Box No. II          Observations where certain claims were found unsearchable (Continuation of item 2 of first sheet)**

This international search report has not been established in respect of certain claims under Article 17(2)(a) for the following reasons:

1. ☒ Claims Nos.: 15
   because they relate to subject matter not required to be searched by this Authority, namely:
   
   ```
   Claim 15 pertains to a method for treatment of a human being by therapy
   and thus relates to a subject matter on which it is not required to carry
   out an international search.
   ```

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III          Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.    Consequently, this international search report is restricted to the invention first mentioned in the claims; it is covered by claims Nos.:

**Remark on Protest**          ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090062118 A
- WO 2006025371 A
- WO 2006004067 A
- US 5151350 A
- WO 2011118699 A
- WO 2010064437 A
- JP 2008279366 A
- JP 2012198352 A
- WO 200600406 A
- WO 2010095673 A

**Non-patent literature cited in the description**

- Annals of the New York Academy of Sciences. 2005, vol. 1051, 635-646
- *American Heart Journal,* 2006, vol. 152 (4
- *Journal of Chromatography A,* 2010, vol. 1217, 1298-1304
- *ANALYTICAL BIOCHEMISTRY,* 1990, vol. 191, 343-346